# EUROPEAN PATENT APPLICATION

(11) **EP 2 116 602 A1**
(43) Date of publication of application: **11.11.2009**
(21) Application number: 08305158.1
(22) Date of filing: 07.05.2008
(51) Int. Cl.: C12N 15/11, A61K 31/713

(54) **Combination products for treating cancer**

(71) Applicant: Institut Gustave Roussy, F-94800 Villejuif (FR)
(72) Inventor: Busson, Pierre, 92160, Antony (FR); Friboulet, Luc, 92160, Antony (FR); Durieu, Catherine, 92170, Vanves (FR)
(74) Representative: Cabinet Plasseraud

(57) **Abstract**

The invention relates to the field of cancer, preferably to nasopharyngeal carcinoma (NPC), and in particular to new products, compositions, plasmid vector and methods for cancer therapy.

The present invention relates in particular to products containing:
(iii) at least one antagonist of c-IAP2 (cellular inhibitor of apoptosis protein 2), and
(iv) at least one agonist of TLR3 (Toll Like Receptor 3),

as a combined preparation for simultaneous, separate, or sequential use for treating a cancer expressing c-IAP2, preferably nasopharyngeal carcinoma (NPC), in a subject.

## Description

### Field of the invention

The invention relates to the field of cancer, preferably to nasopharyngeal carcinoma (NPC), and in particular to new products, compositions, plasmid vector and methods for cancer therapy.

### Background of the Invention

Nasopharyngeal carcinoma (NPC) is a malignant tumor arising from the epithelial lining of the nasopharynx. NPC is consistently associated to the Epstein-Barr virus and is the third most frequent virus-associated malignancy in humans [10, 11]. High incidences foci (25 per 100,000 persons a year) are localized in South East Asia, especially in South China. There are also large areas of intermediate incidence (about 3 to 8 per 100,000 a year) including North and Eastern Africa. NPC has a complex multifactorial etiology involving dietary factors and genetic predisposition in addition to EBV-infection [12, 13]. The malignant phenotype of NPC cells result from patent EBV infection combined to genetic, epigenetic and functional cellular alterations [10]. Most viral genes are silent in NPC cells but a number of them are consistently expressed such as short untranslated RNAs called EBERs 1 and 2, one nuclear protein called EBNA1 (Epstein-Barr Nuclear Antigen 1) and two membrane protein called LMP1 and 2 (Latent Membrane Proteins 1 and 2). Very high amounts of EBERs are produced in the nuclei of NPC cells; a fraction of these molecules are exported in the cytoplasm [14]. In addition to latent EBV infection, the malignant phenotype of NPC cells requires a number of genetic alterations. The most common alterations are tumor suppressor gene inactivations at the *INK*/*ARF* (9p21.3) and *RASSF1A* (3p21.3) loci [15]. Amplification and overexpression of the *CCND1* gene are also frequently observed in NPC specimens [16].

Although NPC treatment has been significantly improved in the recent years, it remains a very serious disease due to the high frequency of distant metastases even in the case of small primary tumors and the frequency of long term secondary effects [17].

Consequently, there is an urgent need for therapies to treat NPC, and generally cancer, which therapies are more effective than current regimens.

### Summary of the invention

In a first aspect, the present invention relates to products containing:
(i) at least one antagonist of c-IAP2 (cellular inhibitor of apoptosis protein 2), and
(ii) at least one agonist of TLR3 (Toll Like Receptor 3),
   as a combined preparation for simultaneous, separate, or sequential use for treating a cancer expressing c-IAP2, preferably nasopharyngeal carcinoma (NPC), in a subject.

In a second aspect, the present invention relates to a method for treating a cancer expressing c-IAP2, preferably nasopharyngeal carcinoma (NPC), comprising the step of simultaneously, separately, or sequentially administrating to a subject in need thereof a therapeutically effective amount of:
(i) at least one antagonist of c-IAP2 (cellular inhibitor of apoptosis protein 2), and
(ii) at least one agonist of TLR3 (Toll Like Receptor 3).

In a third aspect, the present invention relates finally to a pharmaceutical composition comprising:
(i) at least one antagonist of c-IAP2 (cellular inhibitor of apoptosis protein 2), and
(ii) at least one agonist of TLR3 (Toll Like Receptor 3), and
(iii) optionally, a pharmaceutically acceptable carrier.

### Brief description of the drawings

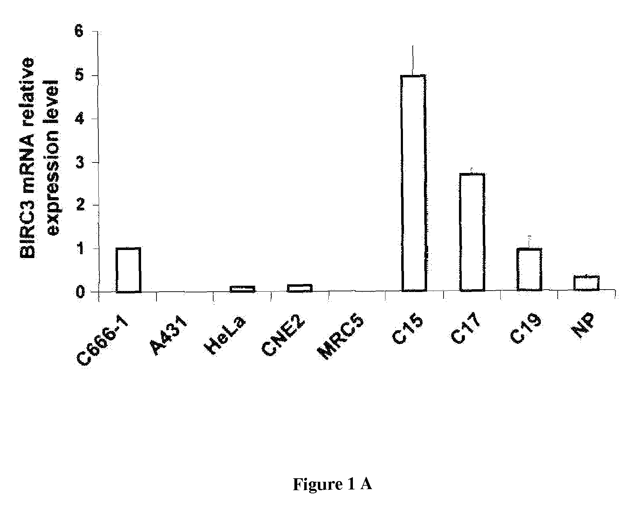

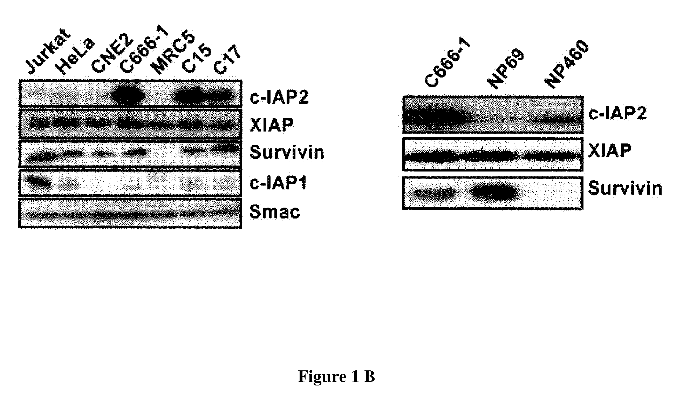

The figure 1 shows the recurrent overexpression of the c-IAP2 protein in NPC tumor lines and clinical specimens despite hemizygous losses of *BIRC3.* Figure 1A, assessment of *BIRC3* gene expression by real time reverse-PCR. Amounts of *BIRC3* transcripts were deduced from Ct values and normalized with values obtained from GADPH RNA amplifications. Relative levels of expression are referred to the value obtained for C666-1 NPC cells arbitrarily set at one. To make accurate comparisons with NPC xenografts, CNE2, Hela and A431 cells were grown as xenografts prior to RNA extraction. Human untransformed MRC5 fibroblasts which are sensitive to contact inhibition were collected as quiescent cells after 3 days of growth arrest resulting from cell confluence.

Figure 1B, *right panel,* western blot analysis of c-IAP2, XIAP, c-IAP1, Survivin and Smac in NPC and non-NPC cell lines. *Left panel,* western blot analysis of IAP proteins in immortalized non-tumorigenic nasopharyngeal epithelial cells: NP69 (SV40 infection), NP 460 (telomerase transfection).

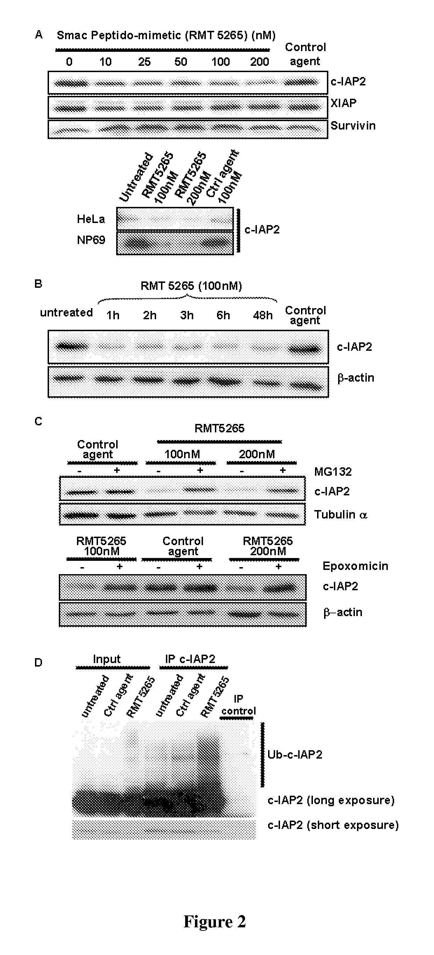

The figure 2 shows the proteasome-dependent degradation of c-IAP2 induced by RMT5265. *A, upper panel,* selective and dose-dependent decrease in c-IAP2 concentration under treatment of C666-1 cells by RMT5265 or control compound (HS4044). Cells were collected after 48h of treatment. Note that XIAP and survivin concentrations are not significantly affected. *Lower panel,* a similar decrease in c-IAP2 concentration is induced by RMT5265 in Hela and NP69 cells. *B,* kinetics of the decrease in c-IAP2 concentration induced by RMT5265 in C666-1 cells. *C*, the decrease in c-IAP2 concentration induced by RMT5265 is prevented by pre-incubation (15 min) and concomitant incubation with the proteasome inhibitors MG132 or epoxomycin. *D*, RMT5265 increases the amount of ubiquitinated forms of c-IAP2 detected in C666-1 cells. In order to increase the signal to noise ratio in the detection of all forms of c-IAP2, they were immunoprecipitated prior to western blot analysis. Treatment by RMT5265 enhanced the detection of a ladder of minor c-IAP2 species with a spacing of about 8 kDa highly suggestive of a polyubiquitination process.

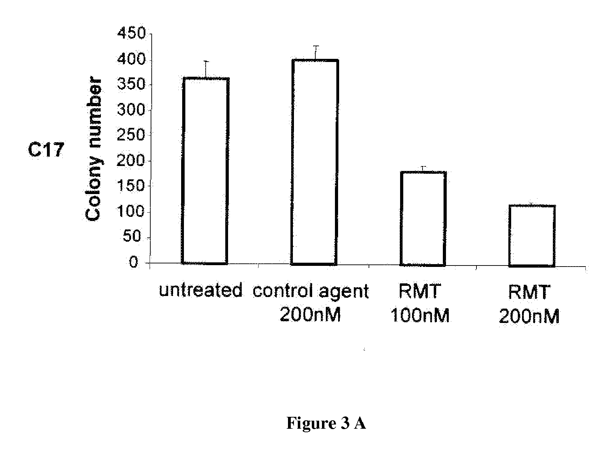

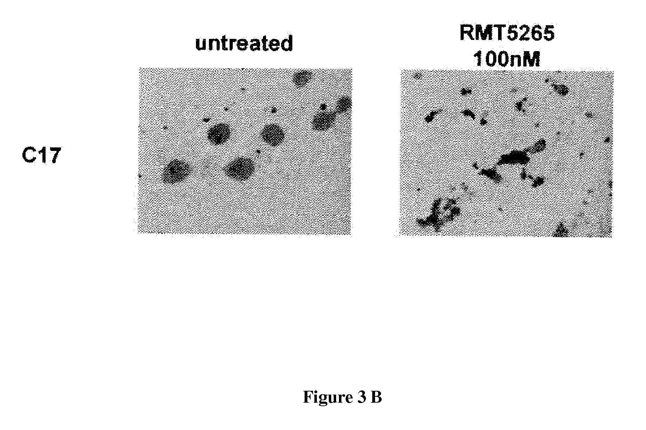

Figure 3 shows the selective inhibitory effects of RMT5265 on NPC cells growth *in vitro.* Figure 3A and 3B, short term colony assay performed on C17 NPC cells treated by RMT5265, control agent (HS4044) or untreated. Cells obtained by dispersion of C17 xenografts were seeded at day 1 at high density (1,2.10⁶ cells per well in 24 wells plate) and treated by pharmacological agents from day 4 to 7. Cell colonies were stained with rhodamine.

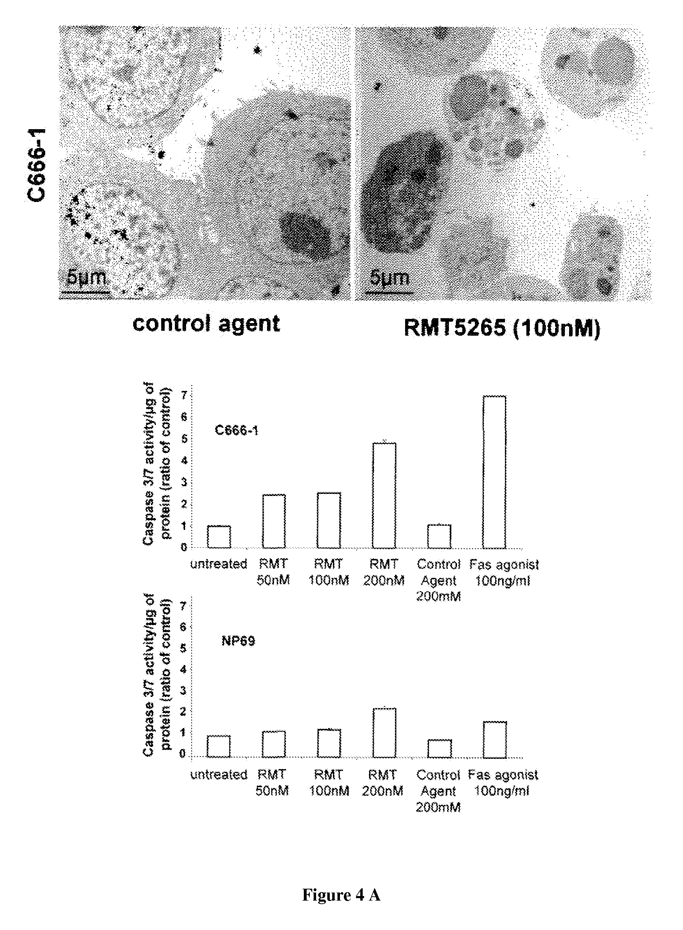

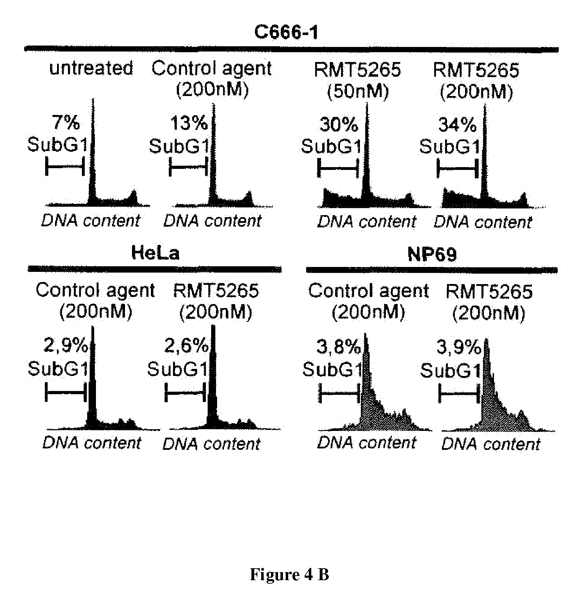

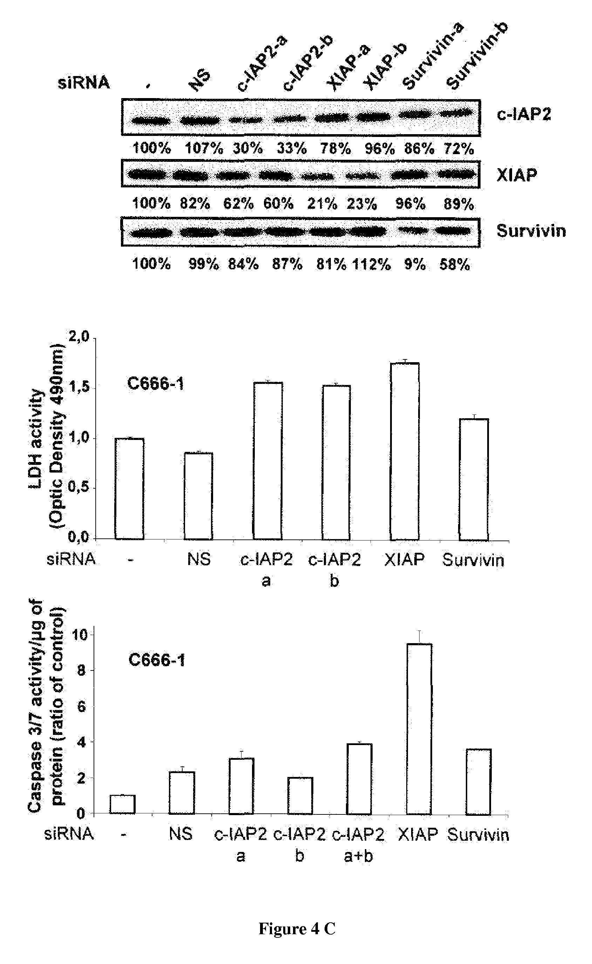

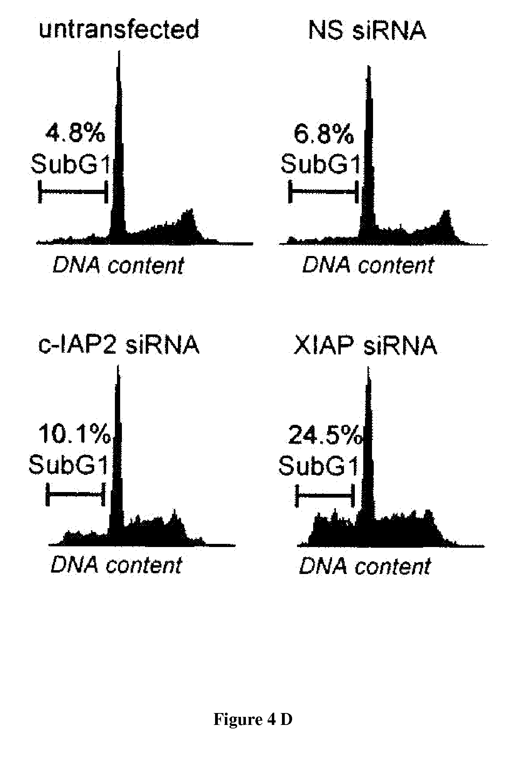

Figure 4 shows the short term effects of c-IAP2 depletion in C666-1 NPC cells. *A, upper panel,* electron microscopy of C666-1 cells treated by RMT5265 or control agent. Cell preparation and observations were done as previously described [31]. *Lower panel,* chemiluminescent assay of caspases-3/7 activity in extracts of NPC cells (C666-1) and immortalized nasopharyngeal epithelial cells (NP 69) treated with RMT5265 for 48 h. Cells treated with a Fas agonist antibody for 6 hours provided a positive control for C666-1 cells. *B,* flow-cytometry assessment of DNA content and counting of cells with sub-G1 characteristics. RMT5265 increases the fraction of sub-G1 cells in NPC cells (C666-1) but not in non-NPC carcinoma cells (Hela) or immortalized nasopharyngeal epithelial cells (NP69). *C, upper panel,* impact of siRNAs on the cell concentration of c-IAP2, XIAP and survivin (western blot detection; specific protein bands were quantified as explained in the Materials and Methods section). *Lower panel,* assessment of LDH release and caspases-3/7 activity following knock-down of the various IAPs in C666-1 cells. Cells underwent two rounds of siRNA transfection at 48h interval. Cell collection and protein extraction for the LDH and caspase assays were done 48h following the second transfection. Each measurement was done in triplicate. *D*, flow-cytometry assessment of DNA content and counting of cells with sub-G1 characteristics after siRNA transfection. Except for electron microscopy, all data displayed in this figure are representative of at least 3 similar experiments.

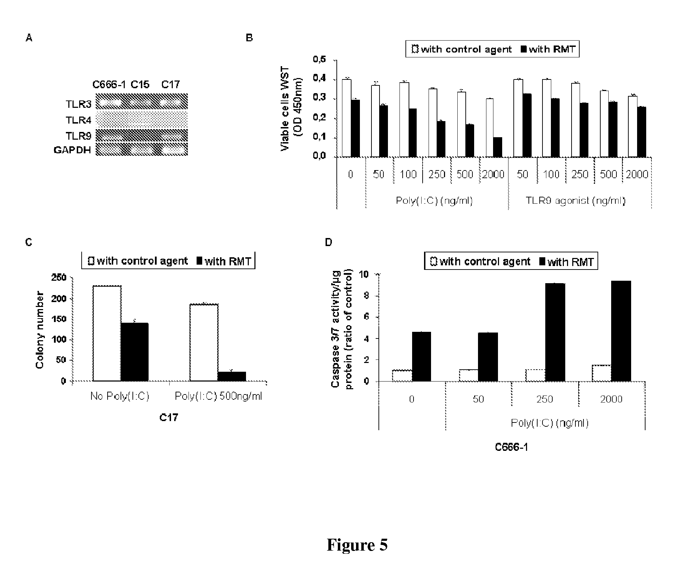

Figure 5 shows the RMT5265 unmasks sensitivity of NPC cells to TLR3 mediated apoptosis. A, transcription of TLR genes assessed by reverse-PCR in three NPC tumor lines. TLR3 (601 bp) and TLR9 (451 bp) amplicons are detected in all 3 lines. Two TLR4 specific amplicons (409 and 242 bp) are detected in C15 but not in C666-1 and C17 RNAs. *B,* short term growth assay of C666-1 cells treated for 72h with various concentrations of TLR3 (poly(I:C)) or TLR9 (CpG) agonists combined to RMT5265 (100 nM) or control agent (100 nM). Amounts of viable cells were evaluated by reduction of WST, a soluble form of MTT, as explained in the Materials and Methods section. Each measurement was done in triplicate. *C*, counting of C17 NPC cell colonies following three days of treatment with poly(I:C) combined to RMT5265 (100 nM) or control combinations. The procedure used for this short term colony assay was the same as in Figure 3B. Colony numbers were the mean of 3 counts made in 3 distinct wells of 24 well plates. *D*, chemiluminescent assay of caspases-3/7 activity in C666-1 cells following 48h of treatment with various concentrations of poly(I:C) combined to RMT5265 or control agent (100 nM). Each measurement was done in duplicate. All data in this figure are representative of at least 3 similar experiments.

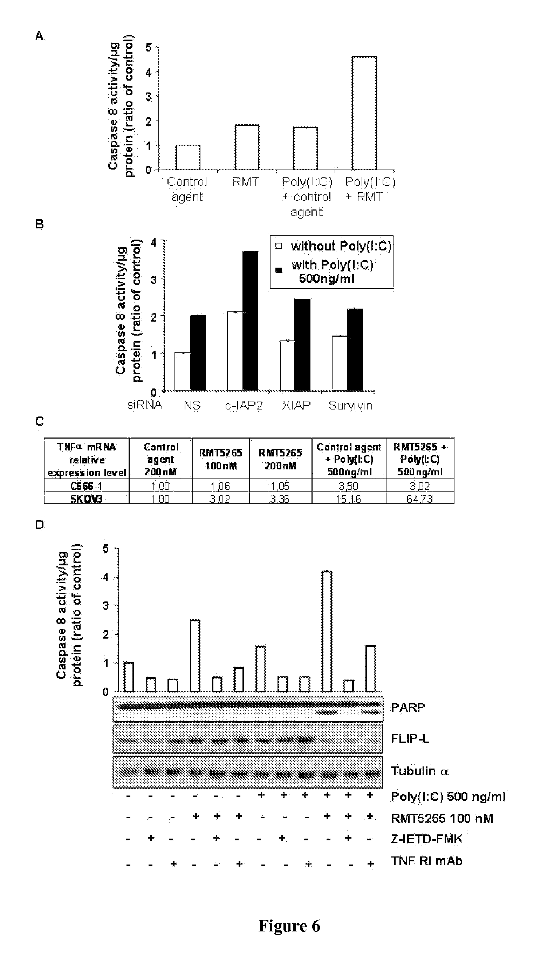

Figure 6 shows the role of caspase-8 activation and TNF-R1 engagement in apoptosis induced by TLR3 stimulation combined to c-IAP2 inhibition. *A,* chemiluminescent assay of caspase-8 activity in C666-1 cells treated for 16h with RMT5265 combined to poly(I:C)(500 ng/ml) or control combinations. Each measurement was done in duplicate. *B,* assay of caspase-8 activity in C666-1 cells transfected with various anti-IAP siRNAs and treated with poly(I:C). Cells underwent two rounds of siRNA transfection at 48h interval. Treatment by poly(I:C) was started 8h after the second transfection and cells were collected 16h later for protein extraction and caspase-8 assay. Each measurement was done in duplicate. *C*, Measurement of TNE-α gene transcription by real time reverse-PCR in C666-1 (NPC) and SKOV3 (ovarian carcinoma) cells treated with RMT5265, poly(I:C) or combination of both for 16h. Amounts of TNE- α transcripts were deduced from Ct values and normalized with values obtained from eF1α RNA amplification. For each cell line, relative levels of expression are referred to the value obtained from cells treated with the control agent (HS4044). *D*, Caspase-8 assay (chemiluminescence) and PARP cleavage assay (western blot) for C666-1 cells treated by various combinations of the following reagents: poly(I:C), RMT5265, a caspase-8 inhibitory peptide (Z-IETD-FMK) (50µM) and an anti-TNF-R1 blocking antibody (10µg/ml). The caspase-8 inhibitory peptide and the anti-TNF-R1 antibody were applied one hour prior poly(1:C) and/or RMT5265. Cells were collected from protein extraction 16h later. PARP cleavage yields a 90 kD truncated form in addition to the intact format 115 kD. All data in this figure are representative of at least 2 similar experiments.

### Description of the preferred embodiments

In order to unveil novel therapeutic targets, it has been undertaken a study based on the basis of a transcriptome analysis of NPC xenografts, whose chromosome alterations had been previously explored, the inventors propose that NPC cells were dependent on a permanent overexpression of the c-iAP2 protein.

An "Inhibitor of Apoptosis (IAP) protein" refers to a polypeptide of the protein family that inhibits caspase activity. All but one of the known IAP proteins share a twofold or threefold repeat of a characteristic sequence motif, the Baculovirus Inhibitory Repeat (BIR;∼70 residues; survivin is a recently discovered human IAP that contains one BIR region). This BIR region contains a number of conserved residues.

The IAPs are a family of anti-apoptotic proteins, conserved across several species and suspected to contribute to a large group of human malignancies [1, 2]. Among them, Two IAPs, c-IAP1 and c-IAP2 are encoded by genes located at close proximity in the 11q22 chromosome band. These genes are frequently detected in malignant cells but their level of expression is highly dependent on tumor origin and histological type. For example, both c-IAP1 and 2 are overexpressed and contribute to rapid tumor growth in human hepatocellular carcinomas whereas in oesophageal carcinomas only c-IAP1 but not c-IAP2 is consistently overexpressed [4, 5].

The inventors have now established that c-IAP2 has a critical role in NPC cell survival and proliferation, as demonstrated by siRNA and pharmacological inhibition.

c-IAP2 protein is coded by BIRC3 (Baculoviral IAP repeat-containing 3) gene.

More specifically, c-IAP2 is essential to antagonize caspase 8 activation, since c-IAP2 inactivation combined with the activation of TLR3 results in a massive apoptosis of tumor cells.

Thus, the inventors have established that the combination of a c-IAP2 antagonist and of a TLR3 agonist can be successfully used for treating nasopharyngeal carcinoma (NPC).

Accordingly, a first object of the present invention is directed to products containing:
(i) at least one antagonist of c-IAP2 (cellular inhibitor of apoptosis protein 2), and
(ii) at least one agonist of TLR3 (Toll Like Receptor 3),
   as a combined preparation for simultaneous, separate, or sequential use for treating a cancer expressing c-IAP2, preferably nasopharyngeal carcinoma (NPC), in a subject.

The term subject refers to a mammal and preferably to a human.

The expression "cancer expressing c-IAP2" refers to a tumor, wherein tumor cells express the c-IAP2 protein. Such tumor can be simply identified by method well known from the skilled person. As an example, such methods include immunohistochemistry or western blot using antibodies directed against the c-IAP2 protein (accession number 2205253B), such as anti-c-IAP2 antibodies available from SANTA CRUZ BIOTECHNOLOGY, INC., ABCAM, ABD SEROTEC, CELL SIGNALING TECHNOLOGY, EVEREST BIOTECH, LIFESPAN BIOSCIENCES, or from NOVUS BIOLOGICALS. As another example, such methods also include c-IAP2 transcript (cDNA accession number AR129833) detection like northern blot or RT-PCR using c-IAP2 specific probes or primers respectively.

Preferably, said cancer expressing c-IAP2 corresponds to a nasopharyngeal carcinoma (NPC).

"Cellular apoptosis" is defined herein as a mechanism of programmed cell death, the most common form of physiological (as opposed to pathological) cell death. Apoptosis is an active process requiring metabolic activity by the dying cell; often characterized by shrinkage of the cell, cleavage of the DNA into fragments that give a so-called "laddering pattern" on gels and by condensation and margination of chromatin. Methodology for measuring apoptosis includes, but is not limited to: measurement of DNA fragmentation by pulsed field gel electrophoresis (Belyaev IY and Harms-Ringdahl M., (2002) Radiats Biol Radioecol. 42: 279-83 [45]) or by terminal deoxynucleotidyl transferase-mediated deoxyuridinetriphosphate nick end-labeling (TUNEL) (Edston E. et al., (2002) Int J Legal Med 116:22-6 [46]), measurement of membrane dielectic changes (Wang X et al. (2002) Biochim Biophys Acta 1564: 412-20), microscopic examination and confirmation of the presence of characteristic pyknotic nuclei, and others.

As used herein, the term "synergistic effect" is intended to mean that the effect achieved with the combination of the compounds of the present invention and either the chemotherapeutic agents or death receptor agonists of the invention is greater than the effect which is obtained with only one of the compounds, agents or agonists, or advantageously the effect which is obtained with the combination of the above compounds, agents or agonists is greater than the addition of the effects obtained with each of the compounds, agents or agonists used separately. Such synergy enables smaller doses to be given.

### Antagonist of c-IAP2

The expression "antagonist of c-IAP2" refers to compounds which inhibit the apoptosis inhibition by cIAP2. Such antagonists of c-IAP2 can be simply identified by the skilled person and include, as an example, siRNA directed against the c-IAP2 transcripts, chemical compounds, or peptidic inhibitors of c-IAP2 as SMAC/diablo protein or mimetic of SMAC/diablo protein.

"SMAC" refers to a mitochondrial polypeptide, which is released together with cytochrome c from the mitochondria in response to apoptotic stimuli. SMAC promotes caspase activation by binding and neutralizing the IAPs. See, e.g. Du et al., Cell 102:33-42 (2000) [48]; Verhagen et al., Cell 102 :43-53 (2000) [49].

siRNA directed against the c-IAP2 transcripts can be simply identify in view of the cDNA c-IAP2 sequence and include, as an example, the following sequences:
SEQ ID N° 3: AAUGCAGAGUCAUCAAUUA,
SEQ ID N° 4: UGAUCUUGUGUUAGACUUA,
SEQ ID N° 5: CUGCCGGAAUUAUUAAUGA,
SEQ ID N° 6: GCAAGAGAACUGAUUGAUA,
SEQ ID N° 7: GAUGCAAUCAUGAUGAAUA,
SEQ ID N° 8: AUACGACUUGUCAUGUGAA,
SEQ ID N° 9: GGUGUGCAUAUAUGUUGAA,
SEQ ID N° 10: UCGAAGGUGUGCAUAUAUG,
SEQ ID N° 11: GGAUAAUGCUAUGUCAGAA,
SEQ ID N° 12: UCUACCAGUGGAAGAACAA,
SEQ ID N° 13: UCAGCUUCAAGACACUUCA,
SEQ ID N° 14: AUACUCCUGUGAUUAAUGC,
SEQ ID N° 15: AGAGGAGACAGUCCUACUG,
SEQ ID N° 16: UGCAGAGUCAUCAAUUAUC,
SEQ ID N° 17: GAACAAUUGCGGAGACUAC,
SEQ ID N° 18: AUUCCGUUAACAACUUGGA,
SEQ ID N° 19: AGAGAACUGAUUGAUACGA,
SEQ ID N° 20: GGUGUGAGUACUUGAUAAG,
SEQ ID N° 21: UACGACUUGUCAUGUGAAC,
SEQ ID N° 22: GCUGAAGCUGUGUUAUAUG,
SEQ ID N° 23: AAUUCCACACACUCAUUAC,
SEQ ID N° 24: UUCCGUUAACAACUUGGAA,
SEQ ID N° 25: GAGGAGACAGUCCUACUGA,
SEQ ID N° 26: CAGCUGCUAUCCACAUCAG,
SEQ ID N° 27: CAAUUGCGGAGACUACAAG,
SEQ ID N° 28: UAUUGCAGCCACUGUAUUC,
SEQ ID N° 29: CUCUCUGCAAGAAGCUGAA,
SEQ ID N° 30: UGUCAGAACACCUGAGACA
SEQ ID N° 31: UUUACCAGGCUUCUACUAAAGCCCA (c1: HSS100563)
SEQ ID N° 32: UUAACUAGAACACUAGAGGGCCAGU (c2: HSS100562)
SEQ ID N° 33: GCUGCAGAUUCGUUCAGAGUCUAAA (c3: HSS100561)

Preferably, siRNA directed against the c-IAP2 transcripts according to the present invention is SEQ ID N° 31, SEQ ID N° 32 and/or SEQ ID N° 33.

"siRNA" refers to small interfering RNAs, that are capable of causing interference and can cause post-transcriptional silencing of specific genes in cells, for example, mammalian cells (including human cells) and in the body, for example, mammalian bodies (including humans). The phenomenon of RNA interference is described and discussed in Bass, Nature 411: 428-29 (2001) [50]; Elbahir et al., Nature 411: 494-98 (2001) [51]; and Fire et al., Nature 391: 806-11 (1998) (52]; and WO 01/75164, where methods of making interfering RNA also are discussed. The siRNAs based upon the sequences and nucleic acids encoding the gene products disclosed herein typically have fewer than 100 base pairs and can be, e.g., about 30 bps or shorter, and can be made by approaches known in the art, including the use of complementary DNA strands or synthetic approaches. The siRNAs are capable of causing interference and can cause post-transcriptional silencing of specific genes in cells, for example, mammalian cells (including human cells) and in the body, for example, mammalian bodies (including humans). Exemplary siRNAs according to the invention could have up to 29 bps, 25 bps, 22 bps, 21 bps, 20 bps, 15 bps, 10 bps or any integer thereabout or therebetween. Tools for designing optimal inhibitory siRNAs include that available from DNAengine Inc. (Seattle, Wash.) and Ambion, Inc. (Austin, Tex.).

One RNAi technique employs genetic constructs within which sense and anti-sense sequences are placed in regions flanking an intron sequence in proper splicing orientation with donor and acceptor splicing sites. Alternatively, spacer sequences of various lengths may be employed to separate self-complementary regions of sequence in the construct. During processing of the gene construct transcript, intron sequences are spliced-out, allowing sense and anti-sense sequences, as well as splice junction sequences, to bind forming double-stranded RNA. Select ribonucleases then bind to and cleave the double-stranded RNA, thereby initiating the cascade of events leading to degradation of specific mRNA gene sequences, and silencing specific genes.

Chemical compounds antagonists of c-IAP2 can be simply identified by the man skilled in the art. It is for example compounds as described in patent application WO 2007/1041632 (page 6 line 1 to page 14 line 2), which compounds are incorporated herein by reference. Those compounds are interfering in the linkage between c-IAP and caspases by the BIR domain of IAP.

Chemical compounds antagonists of c-IAP2 are also described in US 2006/0264379 pages 131 (formula of compound 1 in the table) to 183 (formula of compound 178 in the table), which compounds are incorporated herein by reference.

Peptidic inhibitors of c-IAP2 according to the present invention can be simply identified by the man skilled in the art. It is for example SMAC/diablo protein of sequence SEQ ID N°1 disclosed in patent application US 2006/0019335 incorporated herein by reference.

In some embodiments, the apoptosis-inducing agent is SMAC/Diablo or a SMAC mimetic or agonist. SMAC/Diablo promotes caspase activity by binding Inhibitor of Apoptosis Proteins (IAPs). See, e.g., Du et al., Cell 102:33-42 (2000) [48]; Verhagen et al., Cell 102:43-53, 2000 [49]; U.S. Patent Application No. 2002/0110851. Administration or expression of SMAC in cells is encompassed by the present invention. SMAC fragments, such as the N-terminal peptides of SMAC (e.g., the N-terminal tetra or heptapeptides (Guo et al., Blood 99(9):3419-3426 (2002) [53]; Srinivasula et al., J. Biol. Chem. 275:36152-36157 (2000) [54]), can also be expressed or administered. See, also, U.S. Patent Application 2002/0132786.

Peptidic inhibitors of c-IAP2 according to the present invention can also be a mimetic of SMAC/diablo protein binding to C-IAP2.

Exemplary SMAC mimetics include, e.g., peptides comprising a tetrapeptide that binds the surface groove within the BIR domain of an IAP, including tetrapeptides of the formula X.sub.1X.sub.2X.sub.3X4, wherein X.sub.1 is A, X.sub.2 is V, T, or I, X.sub.3 is P or A and X4 is F, Y, I or V or other SMAC peptides, agonists or peptidiomimetics described in PCT WO 02/26775

In particular, mimetic of SMAC/diablo protein binding to C-IAP2 can be a polypeptide binding to a BIR domain of the c-IAP through its N-terminal IAP binding motif of sequence SEQ ID N°2 disclosed in patent application US 2006/0019335 incorporated herein by reference, or polycyclic C₂-symmetric (40 carbon atoms) compound RMT 5265 which mimics the three-dimensional structure of the SMAC/Diablo N-terminal tetrapeptide, which increase auto-ubiquity of C-IAP [29].

According to a preferred embodiment of the invention, said antagonist of c-IAP2 is selected in the group comprising siRNA directed against the c-IAP2 transcripts, chemical compounds, and peptidic inhibitors of c-IAP2 and combinations thereof, preferably is a mimetic of SMAC/diablo protein and more preferably is compound RMT5265.

### Agonist of TLR3

Toll Like Receptor 3 is a member of the Toll-like receptor (TLR) family which plays a fundamental role in pathogen recognition and activation of innate immunity. TLRs are highly conserved from Drosophila to humans and share structural and functional similarities. They recognize pathogen-associated molecular patterns (PAMPs) that are expressed on infectious agents, and mediate the production of cytokines necessary for the development of effective immunity. The various TLRs exhibit different patterns of expression. This receptor is most abundantly expressed in placenta and pancreas, and is restricted to the dendritic subpopulation of the leukocytes. It recognizes dsRNA associated with viral infection, and induces the activation of NF-kappaB and the production of type I interferons. It may thus play a role in host defense against viruses. TLR3 mRNA sequence is described in NCBI accession number NM_003265, the sequence of which is shown in NO: 1 of US 2006/0110746 which is incorporated herein by reference. TLR3 is described in WO 98/50547 (the disclosure of which is incorporated herein by reference).

It has been demonstrated that the double-stranded RNA receptor is Toll Like receptor 3 (TLR3) (Alexopoulou et al., 2001 [55]). This receptor has been described to be expressed in membranes of dendritic cells and of cells from colic mucosa. The binding of double-stranded RNA to this receptor activates dendritic cells and activates T lymphocytes.

The term "TLR3 agonist" refers to an affinity agent (i.e., a molecule that binds a target molecule) capable of activating a TLR3 polypeptide to induce a full or partial receptor-mediated response. For example, an agonist of TLR3 induces TLR3-mediated signalling, either directly or indirectly.

In preferred embodiments of the invention, a TLR3 agonist is used to treat a patient. TLR3 agonists are well known in the art and suitable TLR3 agonists are available. Further TLR3 agonists, or derivatives or analogs of known TLR3 agonists can be readily identified, made and/or assessed.

The most commonly used TLR3 agonist are nucleic acid based agonists. Thus in preferred aspects, a TLR3 agonist for use according to the present invention are nucleotide or nucleic acid based. Nucleotide or nucleic-acid based compounds can be assessed for their ability to act as an TLR3 agonist using readily available methods. The nucleic acid based TLR3 agonist can be single-stranded or double-stranded or a mixture thereof. The nucleic acid based TLR3 agonist can comprise deoxyribonucleotides, or ribonucleotides or a mixture thereof. The nucleotides can be natural or synthetic, and may be derivatives or analogs of natural nucleotides, such as for example in Kandimalla et al. ((2003) Nucl. Acid. Res. 31(9): 2393-2400) [56].

The particular TLR3 agonist used in the clinical study, the analysis of which was at the origin of the observations on which the present invention is based was a double stranded RNA compound (dsRNA). The specific compound was polyadenylic-polyuridylic acid, i.e., poly(A): poly(U), polyAU or poly A:U. Double-stranded RNA which represents either genomic or life cycle intermediate material of many viruses activates cells through binding to the dsRNA-dependent protein kinase (PKR), a kinase that initiates a complex molecular anti-viral program (Gil, J., Alcami, J., and Esteban, M. 1999. Induction of apoptosis by double-stranded-RNA-dependent protein kinase (PKR) involves the alpha subunit of eukaryotic translation initiation factor 2 and NF-kappaB. Mol Cell Biol 19:4653-4663 [57]). dsRNA was however only recently suggested to act through TLR3 (Alexopoulou, L., Holt, A. C., Medzhitov, R., and Flavell, R. A. 2001. Recognition of double-stranded RNA and activation of NF-kappaB by Toll-like receptor 3. Nature 413:732-738 [58]). It was reported that dsRNA triggers the production of type 1 IFN, and dsRNA has been reported to have promise for certain clinical applications such as anti-viral therapies. A dsRNA compound referred to as Ampligen, for example, has been studied for its ability induce type 1 IFN production and as a consequence to treat viral infection.

Within the context of the present invention, the term "double-stranded RNA" molecule designates any therapeutically or prophylactically effective (synthetic) double-stranded RNA compound. Such compounds are typically active per se, i.e., they do not encode a polypeptide or do not require translation to be active. dsRNA TLR3 agonists can have any suitable length. Preferably, a dsRNA molecule TLR3 agonist has a length of at least about 10 base pairs (bp), 20 bp, 30 bp, 50 bp, 80 bp, 100 bp, 200 bp, 400 bp, 600 bp, 800 bp or 1000 bp. In one aspect the dsRNA molecule is a short dsRNA having a chain length of less than 30 bp, 50 bp, 80 bp, 100 bp or 200 bp. In another embodiment, the dsRNA molecule is a longer dsRNA, but having a chain length of less than 400 bp, 600 bp, 800 bp or 1000 bp. In another embodiment, the dsRNA molecule is a long dsRNA having a chain length of greater than 1000 bp. In one aspect, a dsRNA composition comprises a heterogenous mixture of dsRNA molecules, wherein a plurality of molecules have differing lengths. Preferably the dsRNA molecules have on average a length of at least about 10 bp, 20 bp, 30 bp, 50 bp, 80 bp, 100 bp, 200 bp, 400 bp, 600 bp, 800 bp or 1000 bp. In another embodiment, a dsRNA composition comprises a plurality dsRNA molecules where at least 20%, 50%, 80%, 90% or 98% of dsRNA molecules have a length of at least about 10 bp, 20 bp, 30 bp, 50 bp, 80 bp, 100 bp, 200 bp, 400 bp, 600 bp, 800 bp or 1000 bp. In a preferred embodiment dsRNA composition has a substantially homogenous mixture of dsRNA molecules, where substantially all the molecules do not differ in chain length by more than 30 bp, 50 bp, 80 bp, 100 bp or 200 bp. Average chain length of nucleic acid TLR3 agonists can be determined easily, for example, by gel permeation chromatography.

Previous studies of double-stranded RNA (dsRNA) assessing their ability to be effective interferon inducers suggested that dsRNA agents must possess the secondary structure of a double stranded helix. Other dsRNA agents which have also been shown to be suitable as TLR3 agonist include double-stranded polynucleotides which are not complementary or not perfectly complementary; these have been known as, so-called "mismatched" or "loop-out" structures and exist in naturally occurring RNAs such as transfer tRNAs, ribosomal RNAs and the viral RNA secondary structures. One commonly cited dsRNA compound, Ampligen, comprises a structure where a few parts of cytidine in the poly I:poly C structure are replaced with uridine (i.e. mismatched RNA); this compound has been reported to have physiological activity similar to that of the parent poly I:poly C. However it will be appreciated that TLR3 agonists of any type and configuration can be used in accordance with this invention.

Generally, the polynucleotides need to be resistant to nucleases in order to remain as macromolecules for a sufficient length of time; polynucleotides are less sensitive to nuclease attack when they are in a helical complex. However, certain analogs, such as Ampligen.TM., appear to retain their TLR3 agonist activity.

In a particular embodiment, each strand of these dsRNAs can have a length comprised between about 5 and 50 bases, more preferably between 5 and 40, 35, 30, 25 or 20 bases. Each strand is preferably perfectly complementary to the other. Preferred examples of such dsRNAs are homopolyRNAs, i.e., dsRNAs in which each strand comprises essentially a repeat of the same base; or comprise a homopolyRNA region.

The base may be any naturally occurring base (e.g., polyA, polyU, polyC, polyG) or non- naturally occurring (e.g., chemically synthesized or modified) base (e.g., polyl). Polynucleotides typified by polyinosinic-polycytidylic acid, i.e., poly(I): poly(C) or poly I:C and polyadenylic-polyuridylic acid, i.e., poly(A): poly(U) or poly A:U, are well-known compounds in the art and have been known to induce interferon production by immune cells. Thus in preferred embodiments, the TLR3 agonist for use according to the invention is a double stranded nucleic acid selected from the group consisting of: polyinosinic acid and polycytidylic acid, polyadenylic acid and polyuridylic acid, polyinosinic acid analogue and polycytidylic acid, polyinosinic acid and polycytidylic acid analogue, polyinosinic acid analogue and polycytidylic acid analogue, polyadenylic acid analogue and polyuridylic acid, polyadenylic acid and polyuridylic acid analogue, and polyadenylic acid analogue and polyuridylic acid analogue.

It will be appreciated that nucleic acid-based agonists of TLR3 can be designed using any suitable method. Preferably, the basic requirement of stability and resistance to nuclease attack and the preferences for chain length are taken into account, and that structural changes can be tested and assessed with reference to the a rAₙ:rUₙ or rIₙ:rCₙ complex for example. Measures can be taken to increase stability and resistance to nucleases, or to increase or optionally decrease interferon-inducing action.

Other examples of dsRNA include nucleic acids described in U.S. Pat. Nos. 5,298,614 and 6,780,429. U.S. Pat. No. 5,298,614 reports that when chain length of the double stranded nucleic acid derivatives is limited to certain ranges, the resulting substances exhibit desired physiological activity with markedly less toxicity, providing polynucleotides having a length of about 50 to 10,000 as calculated by base pair numbers. Also described are derivative wherein the purine or pyrimidine ring in the nucleic acid polymer is substituted with at least one SH group, or said derivative contains a disulphide bond, or both (preferred ratio of number of sulphur atoms to cytidylic acid present in the poly C are 1:6 to 39). U.S. Pat. No. 6,780,429 describes a particular type of dsRNA compounds that are "chain-shortened" having lengths of about 100 to 1,000 as calculated by base pair numbers, or preferably from 200 to 800, and more preferably from 300 to 600. The latter compounds are reported to contain low numbers of 2'-5' phosphodiester bonds by a method designed to avoid phosphate groups causing intramolecular rearrangement from 3' position to 2' position through a mechanism called pseudo rotation simultaneously that can occur during hydrolysis of poylnucleotides, resulting in a portion of 3'-5' phosphodiester bonds in the chain-shortened polynucleotide molecule being replaced by 2'-5' phosphodiester bonds. The disclosure of each of these references is incorporated herein by reference.

Other nucleic acid agonists that can be suitable for use as TLR3 agonists are provided in: Field et al.: Proc. Nat. Acad. Sci. U.S. 58, 1004, (1967) [59]; Field et al.: Proc. Nat. Acad. Sci. U.S. 58, 2102, (1967) [60]; Field et al.: Proc. Nat. Acad. Sci. U.S. 61, 340, (1968) [61]; Tytell et al.: Proc. Nat. Acad. Sci. U.S. 58, 1719, (1967) [62]; Field et al.: J. Gen. Physiol. 56, 905 (1970) [63]; De Clercq et al.: Methods in Enzymology, 78, 291 (1981) [64]. A number of synthetic nucleic acid derivatives have been described, including homopolymer-homopolymer complexes (Double Strand Nucleic Acid Polymer such as those in which poly I:C or poly A:U are a parent structure, where these homopolymer-homopolymer complexes contain: (1) base modifications, exemplified by Polyinosinic acid-poly(5-bromocytidylic acid), Polyinosinic acid-poly(2-thiocytidylic acid), Poly(7-deazainosinic acid)-polycytidylic acid, Poly(7-deazainosinic acid)-poly(5-bromocytidylic acid), and Polyinosinic acid-poly(5-thiouridylic acid); (2) Sugar Modifications, exemplified by Poly(2'-azidoinosinic acid)-polycytidylic acid; and (3) Phosphoric Acid Modifications, exemplified by Polyinosinic acid-poly(cytidyl-5'-thiophosphoric acid). Other synthetic nucleic acid derivatives that have been described include interchanged copolymers, exemplified by Poly(adenylic acid-uridylic acid); and homopolymer-copolymer complexes, exemplified by Polyinosinic acid-poly(cytidylic acid-uridylic acid) and Polyinosinic acid-poly(citydylic acid-4-thiouridylic acid). Other synthetic nucleic acid derivatives that have been described include complexes of synthetic nucleic acid with polycation, exemplified by Polyinosinic acid-polycytidylic acid-poly-L-lysinecarboxy-methylcellulose complex (called "Poly ICLC"). Yet another example of synthetic nucleic acid derivative is Polyinosinic acid-poly(1-vinylcytosine).

One example of a TLR3 agonist is Ampligen.TM. (Hemispherx, Inc., of Rockville, Md., U.S.A.), a dsRNA formed by complexes of polyriboinosinic and polyribocytidylic/uridylic acid, such as rIₙ:r(Cₓ,U or G)ₙ where x has a value from 4 to 29, e.g., rIₙ:r(C₁₂ U)ₙ. Many mismatched dsRNA polymers which behave similarly to Ampligen have been studied; mismatched dsRNA based on poly I:C has included complexes of a polyinosinate and a polycytidylate containing a proportion of uracil bases or guanidine bases, e.g., from 1 in 5 to 1 in 30 such bases. The key therapeutic advantage of mismatched dsRNAs over other forms of natural and/or synthetic dsRNAs a reported reduction in toxicity over compounds such as those described in Lampson et al in U.S. Pat. No. 3,666,646.

Specific examples of double-stranded RNA according to the present invention further include Polyadenur (Ipsen) and Ampligen (Hemispherx). Polyadenur is a polyA/U RNA molecule, i.e., contains a polyA strand and a polyU strand. Polyadenur has been developed for the potential treatment of hepatitis B virus (HBV) infection. Ampligen is of a polyI/polyC compound (or a variant thereof comprising a polyI/polyC12U RNA molecule). Ampligen is disclosed for instance in EP 281 380 or EP 113 162. Ampligen has been proposed for the treatment of cancer, viral infections and immune disorders. It was developed primarily for the potential treatment of myalgic encephalomyelitis (ME, or chronic fatigue syndrome/chronic fatigue immune dysfunction syndrome, CFS/CFIDS).

A particular example of a dsRNA for use in the present invention is a dsRNA comprising a polyA/polyU region, wherein each strand of said dsRNA contains less than 25 bases.

Another particular example of a dsRNA for use in the present invention is a dsRNA comprising a polyI/polyC(U) region, wherein each strand of said dsRNA contains less than 25 bases.

Further dsRNAs have been disclosed in the literature or may be developed, which can be used within the present invention. More generally, any synthetic double-stranded homopolyRNA may be used in the context of this invention.

TLR3 agonist can also be any organic or inorganic substance, such as a lipid, peptide, polypeptide, small molecule, etc., in isolated or in mixture with other substances. The TLR3 agonist candidate may be selected from a combinatorial library of products, for instance. In a preferred embodiment, the TLR3 agonist is an antibody directed against TLR3 receptor and which is capable of activating a TLR3 receptor to induce a full or partial receptor-mediated response. The TLR3 agonist can also be an antibody fragment or derivative of an antibody directed against TLR3 receptor and which is capable of activating a TLR3 receptor to induce a full or partial receptor-mediated response.

According to a preferred embodiment of the invention, said agonist of TLR3 is selected in the group comprising single-stranded deoxyribonucleotides, double-stranded deoxyribonucleotides, single-stranded ribonucleotides, double-stranded ribonucleotides and combinations thereof. More preferably, according to the present invention, said agonist of TLR3 is a poly (deoxyinosine-deoxycytosine) (i.e. Poly(I:C)).

In a specific embodiment, the products of the invention further comprise at least one pharmaceutically acceptable carrier.

The phrase "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin.

According to still another specific embodiment, said at least one c-IAP2 antagonist or TLR3 agonist described previously are administrated by intra-tumoral injection.

According to the present invention, an "effective amount" of a composition is one which is sufficient to achieve a desired biological effect, in this case inducing apoptosis in tumor cells. It is understood that the effective dosage will be dependent upon the age, sex, health, and weight of the recipient, kind of concurrent treatment, if any, frequency of treatment, and the nature of the effect desired. The ranges of effective doses provided below are not intended to limit the invention and represent preferred dose ranges. However, the preferred dosage can be tailored to the individual subject, as is understood and determinable by one of skill in the art, without undue experimentation.

In a second aspect, the present invention relates to a method for treating a cancer expressing c-IAP2, preferably nasopharyngeal carcinoma (NPC), comprising the step of simultaneously, separately, or sequentially administrating to a subject in need thereof a therapeutically effective amount of:
(i) at least one antagonist of c-IAP2 (cellular inhibitor of apoptosis protein 2), and
(ii) at least one agonist of TLR3 (Toll Like Receptor 3).

Terms and expression of the second aspect of the present invention are the same that for the first aspect of the invention.

In a third aspect, the present invention relates finally to a pharmaceutical composition comprising:
(i) at least one antagonist of c-IAP2 (cellular inhibitor of apoptosis protein 2), and
(ii) at least one agonist of TLR3 (Toll Like Receptor 3), and
(iii) optionally, a pharmaceutically acceptable carrier.

Terms and expression of the third aspect of the present invention are the same that for the first aspect of the invention.

A pharmaceutical composition of the present invention may be in the form of a solid or liquid. In one aspect, the carrier(s) are particulate, so that the compositions are, for example in tablet or powder form. The carrier(s) may be liquid, with the compositions being, for example, an oral syrup, injectable liquid or aerosol, which is useful in, for example inhalatory administration.

For oral administration, the pharmaceutical composition is preferably in either solid or liquid form, where semi-solid, semi-liquid, suspension and gel forms are included within the forms considered herein as either solid or liquid.

As a solid composition for oral administration, the pharmaceutical composition may be formulated into a powder, granule, compressed tablet, pill, capsule, chewing gum, wafer or the like form. Such a solid composition will typically contain one or more inert diluents or edible carriers. In addition, one or more of the following may be present: binders such as carboxymethylcellulose, ethyl cellulose, microcrystalline cellulose, gum tragacanth or gelatine; excipients such as starch, lactose or dextrins, disintegrating agents such as alginic acid, sodium alginate, Primogel, corn starch and the like; lubricants such as magnesium stearate or Sterotex; glidants such as colloidal silicon dioxide; sweetening agents such as sucrose or saccharin; a flavouring agent such as peppermint, methyl salicylate or orange flavouring; and a coloring agent.

When the pharmaceutical composition is in the form of a capsule, e.g., a gelatine capsule, it may contain, in addition, to materials of the above type a liquid carrier such as polyethylene glycol or oil such as soybean or vegetable oil.

The pharmaceutical composition may be in the form of a liquid, e.g., an elixir syrup, solution, emulsion or suspension. The liquid may be for oral administration or for delivery by injection, as two examples. When intended for oral administration, preferred composition contain, in addition to the present compounds, one or more of a sweetening agent, preservatives, dye/colorant and flavor enhancer. In a composition intended to be administered by injection, one or more of a surfactant, preservative, wetting agent, dispersing agent, suspending agent, buffer, stabilizer and isotonic agent may be included.

The liquid pharmaceutical compositions of the present invention, whether they be solutions, suspensions or other like form, may include one or more of the following adjuvants: sterile diluents such as water for injection, saline solution, preferably physiological saline, Ringer's solution, isotonic sodium chloride, fixed oils such as synthetic mono or diglycerides which may serve as the solvent or suspending medium, polyethylene glycols, glycerine, propylene glycol or other solvents; antibacterial agents such a s benzyl alcohol or methyl paraben; encapsulating agents such as cyclodextrins or functionalized cyclodextrins, including, but not limited to, α, β, or δ-hydroxypropylcyclodextrins or Captisol: antioxidants such as ascorbic acid or sodium bisulfite; chelating agents such as ethylenediamine tetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. An injectable pharmaceutical composition is preferably sterile.

The pharmaceutical composition of the present invention in solid or liquid form may include an agent that binds to the compound of the present invention and thereby assists in the delivery of the compound. Suitable agents that may act in this capacity include, but are not limited to, a monoclonal or polyclonal antibody, a protein or a liposome.

The pharmaceutical composition of the present invention may consist of dosage units that can be administered as an aerosol. The term aerosol is used to denote a variety of systems ranging from those of colloidal nature to systems consisting of pressurized packages. Delivery may be by a liquefied or compressed gas or by a suitable pump system that dispenses the active ingredients. Aerosols of compounds of the present invention may be delivered in single phase, bi-phasic, or tri-phasic systems in order to deliver the active ingredient(s). Delivery of the aerosol includes the necessary container, activators, valves, subcontainers, and the like, which together may form a kit. One skilled in the art, without undue experimentation may determine preferred aerosols.

The pharmaceutical compositions of the present invention may be prepared by methodology well known in the pharmaceutical art. For example, a pharmaceutical composition intended to be administered by injection can be prepared by admixing the compounds of the present invention with sterile, distilled water so as to form a solution. A surfactant may be added to facilitate the formation of a homogeneous solution or suspension. Surfactants are compounds that non-covalently interact with the compounds of the present invention so as to facilitate dissolution or homogeneous suspension of the compounds in the aqueous delivery system.

According to a preferred embodiment, said pharmaceutical compositions of the present invention described previously are administrated by parental route, oral route, intraperitoneal route or intra-tumoral injection, more preferably by parental route.

In the following, the invention is described in more detail with reference to nucleic acid sequences and the examples. Yet, no limitation of the invention is intended by the details of the examples. Rather, the invention pertains to any embodiment, which comprises details which are not explicitly mentioned in the examples herein, but which the skilled person finds without undue effort.

### EXAMPLES

### I- Materials and Methods

### 1) Clinical specimens of NPCs and immunohistology

NPC biopsy fragments were obtained prior to any treatment from 13 NPC patients referred to the Institut Gustave Roussy (9 patients) (France) or the Sfax university hospital (4 patients) (Tunisia). Clinical and pathological characteristics of these tumors are summarized in Supplementary Table 1.

Tumor pieces were fixed in PBS-formaldehyde 4% for 16h and paraffin-embedded. Tissue sections were dewaxed, rehydrated and microwaved at 98°C for 30 min in citrate buffer (10 mM, pH 7.3). After endogenous peroxidase blocking with H₂O₂, slides were incubated with an anti-human c-IAP2 mouse monoclonal antibody (clone F30-2285 from BD Biosciences). Binding of the primary antibody was revealed using the CSA II kit from DAKO (kit based on a tyramide amplification system; DAKOCYTOMATION).

### 2) Xenografts and cell lines

C15, C17 and C19 are EBV-positive NPC xenografts propagated by subcutaneous passages into nude mice [18, 19].

The C15 xenograft was established from the biopsy of a primary nasopharyngeal tumor in a 13-year-old girl born in Morocco. It has typical characteristics of the juvenile form of North African NPCs, especially substantial expression of the EBV-oncoprotein LMP1 [20].

C17 and C19 were established from metastatic lesions in adult patients from France and Italy respectively. Prior to *in vitro* experiments, C17 xenografted tumors were minced and treated with type II collagenase for cell dispersion, as previously reported [18, 26]. Dispersed C17 cells were seeded in RPMI 1640 medium (GIBCO), Hepes 25mM with 7.5% ECS.

C666-1 is an EBV-positive NPC cell line which is derived from a xenograft and currently propagated by *in vitro* culture [18]. The initial biopsy was obtained from a Chinese patient in Hong-Kong [21]. C666-1 cells were continuously grown in the same medium, using plastic flasks coated with collagen I (BECTON-DICKINSON).

Primary cultures of nasopharyngeal epithelial cells were established in Hong-Kong from non-malignant biopsies from the nasopharyngeal region as previously described [22]. Non-tumorigenic immortalized epithelial cell lines were derived from such primary cultures by SV-40 infection (NP69) or stable transfection of the telomerase catalytic subunit gene hTert (NP460) [22, 23]. NP69 and NP460 were grown in KSFM (keratinocyte serum free media) (GIBCO) supplemented with 10% ECS and Bovine Pituitary Extract for NP460 (GIBCO).

CNE2 is an atypical EBV-negative NPC cell line [24]. Non-NPC malignant epithelial cell lines were also used: HeLa (cervix carcinoma), A431 (vulvar epidermoid carcinoma) and SKOV3 (ovarian carcinoma). CNE2, Hela and A431 were grown in DMEM (GIBCO) with 5% ECS, SKOV3 in McCoy supplemented with 2% sodium bicarbonate and 10% FCS.

Jurkat, a malignant T-cell line was used in one experiment.

MRC5 are human untransformed fibroblasts purchased from BIOMERIEUX [25].

All culture media were supplemented with gentamycine 100 µg/ml. For some experiments requiring precise comparisons with C15 and C17 xenografts, CNE2, Hela and A431 cells were grown as xenografts into nude mice (subcutaneous injections of 2 to 5 millions cells).

### 3) RNA extraction and transcriptome analysts

Total RNAs from xenografted tumors and cultured cells were extracted using the TRIREAGENT method (EUROMEDEX) combined to additional purification on RNeasy columns (QUIAGEN). Homogenization of tumor pieces was done by rapid grinding in 1 ml TRIREAGENT using a frosted glass grinder with a conical pestle. Quality of RNA-preparations was assessed using LAB-ON-A-CHIP BIOANALYSER 2000 technology (AGILENT TECHNOIOGIES), based on the 28S/18S ribosomal RNAs ratio. For transcription profiling on microarrays, target c-DNAs were synthetised by incubation of total RNA (25 µg) in a cocktail containing Cy3 or Cy5-dUTP (MEN) and SUPERSCRIPT II reverse transcriptase (LIFE TECHNOLOGIES). Microarray slides were pan-genomic oligo-arrays containing 22 000 60-mer oligos representative of 14 217 genes which were manufactured by AGILENT (ref G 4110A). Labelled targets were hybridized to slides for 16h at 42 °C. After washing, slides were scanned with an AGILENT 2565AA DNA array scanner with a resolution of 10 µtm. Data were analysed using the RESOLVER software.

### 4) Reverse transcription-PCR analysis

Total RNA (1µg) was reverse-transcribed using PROTOSCRIPT FIRST STRAND CDNA SYNTHESIS Kit (NEW ENGLAND BIOLABS). Real time PCR was performed in a 25 µl reaction volume, containing 25ng of cDNA template, 10 pmol of each primer and 12,5 µl of SYBR®-Green master mix (APPLIED BIOSYSTEMS) or TaqMan Universal PCR Master Mix (ROCHE). Quantification of *BIRC3* mRNA which codes for c-IAP2, was carried out using the following oligonucleotides:
* BiRC3-sens: 5'-ATCTGGAGATGATCCATGGG-3' (SEQ ID NO: 31) and
* Bi RC3-antisens: 5'-TGTTCAAGTAGATGAGGG-3' (SEQ ID NO: 32).

Amplified GAPDH RNA was used as endogenous control. Quantification of TNF-α mRNA was carried out using the specific primers and internal probe from APPLIED BIOSYSTEMS (Assay ID HS00174128-ml) with concomitant amplification of EF1α-R RNA used as an endogenous control [2]. The amplification reactions were performed using the APPLIED BIOSYSTEM apparatus, ABI PRISM 7000 SEQUENCE DETECTION SYSTEM. Conventional RT-PCR was used for detection of TLR3, 4 and 9 transcripts using primer pairs from R&D SYSTEMS (RDP 266, 268 and 274 respectively).

### 5) FISH (fluorescent in situ hybridization) analysis

Interphase nuclei and metaphase spreads were prepared following standard procedures from the C666-1 NPC cell line and from the C15, C17 and C19 NPC xenografts. The *BIRC3* DNA probe was derived from the BAC clone RP11-659L4 starting at nucleotide 101,609,259, ending at 101,837,762 (BACPAC RESOURCES). This Bac clone contains the whole BiRC3 gene laying from nucleotide 101,693,404 to 101,713,658 at 11q22.2 (http://www.genome-ucsc.edu). This probe was labelled by random priming with fluorescein 5-dUTP (ABBOT-VYSIS). Then, it was tested by FISH on normal metaphases to ensure accurate hybridization of the BAC on its target chromosome. The same BAC was subsequently hybridized on interphase nuclei and metaphases of the 4 NPC tumor lines according to previously published protocols [28]. After hybridization (at 37°C overnight) the slides were washed, air dried and counterstained with DAPI in antifade solution. The images were acquired with a CCD colour camera using the SMART CAPTURE program (DIGITAL SCIENTIFIC).

### 6) Cell treatments with pharmacological reagents and siRNA

The polycyclic C₂-symmetric (40 carbon atoms) compound RMT5265 which mimics the tridimensionnal structure of the Smac/Diablo N-terminal tetrapeptide has been previously described [29]. HS4044 which has a similar structure except acetylation of a critical alanine group was used as a negative control [29]. Both reagents were solubilized in DMSO. Caspase-8 inhibition was realized using Z-IETD-FMK (N-1830 BACHEM) at 50µM. TNF-R1 neutralization was achieved using a monoclonal anti-human TNF-R1 antibody (clone 16803 R&D systems) at 10µg/ml. Proteasome inhibition was performed with MG132 (Calbiochem UK) at 10µM for 15min and Epoxomicin (Calbiochem UK) at 1µM for 15min. As a positive control for apoptosis induction, cells were treated with the Fas agonist monoclonal antibody 7C11 at 100 ng/ml for 6h (immunotech - Beckman-Coulter). Agonists for TLR3 (poly(I:C)) and TLR9 (type C CpG) were from InvivoGen. Transient transfections of siRNA were performed on C666-1 cell at 30-50% confluence in six-well plates. Double-stranded RNA ologonucleotides directed towards c-IAP2 (a: HSS100562, b: HSS100561), XIAP (a: HSS100566, b: HSS100565), Survivin (a: HSS141243, b: HSS141245) and a negative control (1390109) were purchased from invitrogen. Transfections were carried out using Oligofectamine according to manufacturers instructions (Invitrogen). Two successive rounds of siRNA transfection at 48h interval were required for sufficient depletion of c-IAP2.

### 7) Cell growth and viability assays

Clonal cell growth of C666-1 NPC cells was assessed using a feeder layer of Swiss 3T3 murine fibroblasts as previously described [30]. Briefly, irradiated Swiss 3T3 cells kindly provided by T. Magnaldo (Institut Gustave-Roussy Villejuif France) were plated in 6-wells plates (1.5x10⁵/well). Following fibroblast attachment, epithelial cells were added at a density varying from 500 (immortalized NP 69 cells) to 5000/wells (C666-1 NPC cells). Cultured cells were fed each week with fresh medium containing appropriate pharmacological agents. Two to 4 weeks later, cell colonies were stained with a methanol solution of crystal violet. Alternatively, C666-1 cell viability was assayed in a short term assay based on reduction of WST (a soluble form of MTT, Roche Molecular) as described [18]. For this assay, cells were seeded in 96 well plates at 3 X 10⁴ /well. The WST reaction was performed after 72h of culture. *In vitro* growth of C17 NPC cells was evaluated by a short term colony assay. Dispersed cells derived from xenografted tumors were seeded in 24 well plates at 106 cells/well in 1.5 ml in RPMI medium with 20% FCS. The culture medium was changed twice, 16h and 72h later. At the second change of medium, the percentage of ECS was lowered to 7.5%. During the next 3 days, scattered colonies of epithelial cells became apparent on a layer of murine fibroblasts. These colonies were stained with a PBS-formaldehyde solution of rodhamin and counted using an inverted microscope.

### 8) Assessment of cell death and apoptosis

Evaluation of total cell death was performed by colorimetric assay of LDH released in the culture medium with LDH Cytotoxicity Detection Kit (Clontech). Apoptosis was quantitatively assessed by measurement of sub-G1 DNA content. Ethanol-fixed cells were stained with propidium iodide and analysed using a Becton Dickinson Facscalibur and the CellQuest-pro software. Caspases-3/7 and 8 activities were measured using the Caspases-Glo 3/7 and Caspases-Gio 8 Assay kit respectively (Promega). These assays are based on the cleavage of luminogenic substrates containing the amino-acid sequences Z-DEVD and Z-LETD, respectively.

### 9) Cell protein extraction and western blot analysis

Proteins from cultured cells were extracted by lysis in RIPA buffer (50 mM Tris, 150mM NaCl, 5mM EDTA, 0.5% NaDOC, 0.5% NP40, 0.1% SDS) supplemented with a protease inhibitor cocktail (Complete - Roche Diagnostics GmbH, Germany). For protein extraction from xenografts, tumor pieces were crushed in RIPA buffer-0,1% SDS. After sonication on ice, insoluble material was removed by centrifugation at 10, 000 x g for 15 min at 4°C. Protein concentrations were assayed by the Lowry method (Bio-Rad microassay system). Proteins were separated by SDS-PAGE and transferred to PVDF membranes (Immobilon Millipore) for 1h30 at 90V or overnight at 45V at 4°C. Antibodies used for western blotting were mouse monoclonal antibodies directed to human c-IAP2, XIAP and c-IAP1 from BD Biosciences (respective references: 552782, 610763 and 556533). Other mouse monoclonals were specific of Smac/Diablo (Cell Signaling, ref. 9494), Survivin (Santa Cruz, ref. 17779), poly(ADP-ribose) polymerase (PARP; clone C-2-10) (Santa Cruz, ref. 53643). Following incubation with a secondary peroxidase-conjugated antibody chemiluminescent detection was carried out using the Immobilon Western Chemiluminescent HRP Substrate (Milhpore Corporation). When required, blotted membranes were stripped with the Re-Blot Plus Mild Antibody Stripping Solution (Chemicon International Inc.) for 20 min at room temperature. In one instance, immunoprecipitation of c-IAP2 was performed prior to western blot analysis using magnetic beads as previously described [31]. Quantitative assessment of the specific bands was done by film densitometry using a GS-710 calibrated imaging densitometer with the Quantity One software (Biorad, Marnes la Coquette, France). The amount of the corresponding protein was estimated according to the densitometric "volume" of each band.

### II- Results

### 1) Abundant transcription of the BIRC3 gene in NPC cells even when affected by hemizygous deletions

Microarrays containing 22 000 nucleotides representative of 14 217 genes were used to profile gene transcription in 2 NPC xenografts (C15 and C17) against a reference provided by untransformed quiescent human fibroblasts (MRC5).

Subsequent investigations were focused on genes transcribed at a very high level in each xenograft. The main characteristics of the top-10 most transcribed genes - including their chromosome location - are provided in Table 1.

**Table 1**

| Gene symbols (HUGO nomenclature) | Common designation of the gene product | Chromosome location | Micro-array analysis (NPC xenograft/MRC5,- fold change with p value) | | SAGE analysis (ratio of tag numbers) |
|---|---|---|---|---|---|
| | | | C15/MRC5^{a} | C17/MRC5 | C666-1 tumor line / normal NP^{b} |
| TACSTD1 | Tumor-associated calcium signal transducer 1 | 2p21 | 28 | 74 | |
| SCYA20 | CCL20 chemokine | 2q33-37 | 27 | 23 | 32/0 |
| PCOLCE2 | Procollagen C-endopeptidase enhancer_2 | 3q21-24 | 24 | 21 | 1/0 |
| CSTA | Cystatin A | 3g21 | 65 | 70 | |
| FAT10 | Ubiquitin D or diubiguitin | 6p21 | 47 | 69 | 0/0 |
| HLA-DRB5 | | 6p21.3 | 18 | 18 | |
| HLA-DRA | | 6p21.3 | 16 | 24 | |
| HLA-DQB1 | | 6p21.3 | 21 | 22 | |
| EPHX2 | Cytosolic epoxide hydroxylase | 8p21-p12 | 22 | 19 | |
| ALDH1A1 | Aldehyde dehydrogenase 1 family, member Al | 9q21.13 | 20 | 30 | |
| BIRC3 | c-IAP2 | 11q22 | 36 | 33 | 3/0 |
| OCA2 | Melanocyte specific transporter | 15q11.2-q12 | 32 | 45 | |
| PLA2G3 | Secreted phospholipase A2, group III | 22q11.2q13.2 | 16 | 28 | |

| | | | | | |
|---|---|---|---|---|---|
| a: Human untransformed MRC5 fibroblasts were collected in a state of quiescence after 3 days of growth arrest resulting from cell confluence. b: Normal NP: RNA from primary cultures of non-malignant epithelial nasopharyngeal carcinoma cells | | | | | |

*BIRC3* encoding the c-IAP2 protein was one of these genes. Its consistent overexpression was confirmed by quantitative RT-PCR. As shown in Figure 1A, *BIRC3* transcripts were much more abundant in EBV-positive NPC tumor lines (either xenografted or propagated *in vitro*) - C15, C17, C19 and C666-1 - than in any other cell type including untransformed MRC5 fibroblasts, non-NPC malignant epithelial cells (Hela, A431), atypical EBV-negative NPC cells (CNE2) and a primary culture of nasopharyngeal epithelial cells (NP) from normal mucosa. Meanwhile it has been noticed that *BIRC3* was mapping to a chromosomal band (11q22) frequently affected by hemizygous deletions in NPCs as well as in some other tumor types [19, 21, 32]. According to previous studies, the 11 q22 band was known to be affected by hemizygous losses in C17, C19 and C66 but not in the C15 tumor line [19, 21]. Therefore *BIRC3* was expected to be reduced to one copy in C17, C19 and C66 but not in C15 cells. This was formally established by Fish analysis using a BAC probe encompassing the *BIRC3* gene.

FISH detection of the *BIRC3* gene on interphase and metaphase nuclei of NPC cells shows that three of four NPC tumor lines (C666-1, C17 et C19) have alterations of chromosome 11 q resulting in a deletion of one copy of the *BIRC3* gene as shown by the detection of a single spot on interphase nuclei. In contrast the cells from the C15 xenograft retain 2 copies of the *BIRC3* gene. Hybridization on chromosome spreads was obtained for 3 tumor lines. Consistent with observations on interphase nuclei, C666-1 and C17 cells display *BIRC3* spots linked only to one chromosome 11 (with a double spot due to the presence of two chromatides following completion of "S" phase). In contrast C15 cells contain BIRC3 spots linked to 2 chromosomes 11 (each chromosome containing a double spot).

This supported the conclusion that the reduction of the *BIRC3* gene to one copy was not accompanied by a significant decrease in its level of transcription which remained in the same order of magnitude as for the C15 xenograft. This point was even more striking in light of the reduced level of transcription of most other genes mapping to chromosome 11q.

Parallel exploration of chromosome 11 structural alterations and regional overall transcriptional activity in NPC xenografts has been made: quantitative structural chromosome alterations were analyzed by comparative genomic hybridisation (CGH) of genomic DNA from C15, C17 and C19 NPC xenografts. In parallel overall chromosome transcriptional activity were assessed by comparative expressed sequence hybridization (CESH) using total c-DNA derived from the same tumors. Regional overall transcription is at a very low level in the long arm of chromosome 11 (C17) or its distal portion (11q22-24
- C19) which are affected by hemizygous genomic deletions. In contrast the C15 xenograft
- which has no visible chromosome 11 losses - retains a higher overall level of transcription from the 11q. As shown in Table 3, despite their overall low level of transcription at 11q22, the C17 and C19 xenografts exhibit a very high level of *BIRC3* expression. Comparative genomic hybridization (CGH) and comparative expressed sequence hybridization (CESH) was performed as described [19, 44].

Data of expression levels of genes mapping in proximity to *BIRC3* in the 11q22 bands of the C15 and C17 xenografts (coordinates +/- 6Mb) are provided in table 2.

**Table 2**

| Gene name | | Location in Mb | C15 | C17 |
|---|---|---|---|---|
| HSPC148 | AAF29112.1 | 96,209208 | 1,08131488 | 0,676544212 |
| FLJ10251 | BAA91508.1 | 96,2395115 | 1,09950522 | 0,992851469 |
| SRP46 | AAH06181.1 | 96,308905 | 0,99770528 | 0,625586487 |
| KIAA0092 | BAA07654.1 | 97,052564 | 1,1055832 | 0,948766603 |
| MTMR2 | Q13614 | 97,119483 | 0,86580087 | 0,586544665 |
| HSPC048 | AAF29020.1 | 97,6303575 | 1,03831378 | 1,026377912 |
| JRKL | AAB65833.1 | 97,632951 | 1,02406554 | 0,581564408 |
| AD031 | AAG44547.1 | 102,220849 | 1,19574315 | 1,258653241 |
| PGR | P06401 | 102,312003 | 1,94893783 | 1,857010214 |
| TRPC6 | Q9Y210 | 102,7456265 | 0,87527352 | 0,723431961 |
| MGC13040 | AAH06128.1 | 103,293282 | 0,82081589 | 3,397893306 |
| YAP1 | P46937 | 103,399847 | 0,52723151 | 0,364750511 |
| BIRC3 | Q13489 | 103,558587 | 35,971223 | 32,78688525 |
| BIRC2 | Q13490 | 103,591515 | 1,92826841 | 0,6792094 |
| PORIMIN | AAG21694.1 | 103,65242 | 1,09673174 | 1,02501025 |
| MMP7 | P09237 | 103,753462 | 14,0252454 | 2,090738031 |
| MMP20 | 060882 | 103,828917 | 0,80866893 | 2,303616678 |
| MMP27 | AAG28453.1 | 103,926534 | 0,97713504 | 1,185536455 |
| MMP8 | P22894 | 103,9465955 | 0,9938382 | 0,919117647 |
| MMP10 | P09238 | 104,0033855 | 1,14416476 | 1,333155579 |
| MMP1 | P03956 | 104,021869 | 0,02890683 | 0,01 7660387 |
| MMP3 | P08254 | 104,0675345 | 0,87912088 | 0,750243829 |
| MMP12 | P39900 | 104,096672 | 0,94206312 | 0,897907875 |
| MMP13 | P45452 | 104,1771875 | 1,56788962 | 1,301913813 |
| MGC2714 | AAH04169.1 | 104,304871 | 1,5037594 | 1,054407423 |
| FLJ11756 | BAB13905.1 | 104,5942515 | 0,31289111 | 0,113396684 |
| SCDGF-B | AAG39287.1 | 105,2630335 | 1,04090767 | 0,282877429 |
| CASP4 | P49662 | 106,1831025 | 1,0757315 | 0,256963717 |
| CASP5 | P51878 | 106,228993 | 1,12321689 | 0,317611561 |
| CASP1 | P29466 | 106,257377 | 0,50344862 | 0,486807516 |
| ICEBERG | P57730 | 106,3663315 | 0,87047354 | 1,159554731 |
| GRIA4 | P48058 | 107,0227485 | 0,88028169 | 0,872219799 |
| I_1152106 | AK055247.1 | 107,2922695 | 0,7800312 | 0,109471472 |
| AASDHPPT | AAH16728.1 | 107,315499 | 0,9979044 | 0,623441397 |
| GUCY1A2 | CAA90393.1 | 108,080185 | 1,06814783 | 0,894374385 |
| SLN | 000631 | 108,937086 | 0,94948728 | 1,233958539 |
| HSNOV1 | BAB14394.1 | 109,0522155 | 2,08768267 | 2,742731761 |
| CUL5 | Q93034 | 109,285352 | 0,84530854 | 0,757002271 |
| ACAT1 | P24752 | 109,361836 | 0,91207588 | 0,369494531 |
| NPAT | CAA65824.1 | 109,4172765 | 1,12905047 | 0,958588957 |
| ATM | Q13315 | 109,516219 | 0,78740157 | 0,290065265 |

These observations suggested that overexpression of *BIRC3* provided a selective advantage to NPC cells even at a late stage of tumor progression (for example in C17 and C19 xenografts which were derived from metastatic cells).

### Recurrent overexpression of the c-IAP2 protein in NPC tumor lines and clinical specimens

To confirm that intense transcription of *BIRC3* resulted in a high level of c-IAP2 protein expression in NPC cells, three NPC tumor lines were analyzed by western blotting in combination with several references including non-NPC malignant epithelial cells (xenografted Hela cells), atypical EBV-negative NPC cells (xenografted CNE2 cells), non-malignant human fibroblasts grown *in vitro* (MRC5) and non-malignant nasopharyngeal epithelial cells immortalized by 5V40 (NP69) or telomerase gene transfection (NP 460) (Figure 1B). Data were consistent with the observations made at the transcriptional level. A very high amount of c-IAP2 protein was specifically recorded in NPC tumor lines where it was about ten times more abundant than in non-NPC xenografts, MRC5 cells and immortalized nasopharyngeal cells. In contrast, XIAP was equally abundant in all cell types including non-malignant MRC5. Survivin was at an equally high level of expression in NPC and non-NPC tumor lines contrasting with a low level in MRC5 cells. c-IAP1 was abundant in the Jurkat T-cell line used as a positive control whereas it was at a very low level in other cell types, including NPC tumor lines. c-IAP2 expression was also analyzed in tissue sections of clinical NPC specimens. A high level of expression was observed in 11 out of 13 biopsies.

Detection of c-IAP2 by immunohistochemistry in tissue sections of NPC specimens using the F30-2285 monoclonal antibody shows that C666-1 cells grown as a xenografted tumor showing intense cytoplasmic expression consistent with western blotting data displayed in figure 1C. It also shows that Hela cells grown as a xenografted tumor; no c-IAP2 staining is visible although a faint band was detected by western blotting which is more sensitive than immunohistochemistry. Finally it shows that cytoplasmic staining of c-IAP2 in biopsies 4 and 7 respectively; note the intense expression in malignant cells contrasting with a very low expression in stromal cells.

Data of detection of the c-IAP2 protein by immunohistochemistry on tissue sections of NPC biopsies are provided in table 3. Pictures of cases 4 and 7 are displayed in Figure 3B.

**Table 3**

| MO: absence of extra-nodal metastases. M+: presence of extra-nodal metastases at the time of diagnosis. Staining assessment: ++ strong staining of all malignant cells; | | | | | |
|---|---|---|---|---|---|
| Case Number | Origin | Sex/Age | Clinical Staging | Histological subtype | c-IAP2 staining |
| 1 | Portugal | M/50 | T4N2M0 | Type III | + |
| 2 | Tunisia | M/58 | T1N2M0 | Type III | ++ |
| 3 | Morocco | M/59 | T3N1M0 | Type III | + |
| 4 | Tunisia | M/71 | T2NOM0 | Type II | ++ |
| 5 | France | M/55 | T3N3MO | Type II | + |
| 6 | Malaysia | M/47 | T2N0MO | Type III | + |
| 7 | Algeria | M/58 | T3N2MO | Type III | + |
| 8 | Algeria | F/33 | T2N2MO | Type II | + |
| 9 | Algeria | M/72 | T2N2MO | Type III | +/- |
| 10 | Tunisia | M/47 | T3N2M+ | Type II | ++ |
| 11 | Tunisia | F/55 | T4N3M+ | Type II | - |
| 12 | Tunisia | M/38 | T2N2M0 | Type III | ++ |
| 13 | Tunisia | M/40 | T2N3M0 | Type III | ++ |

| | | | | | |
|---|---|---|---|---|---|
| + moderate staining of the majority of malignant cells; +/- faint staining of a minority of malignant cells; - absence of staining. | | | | | |

Its distribution was essentially cytoplasmic with only rare examples of nuclear localization. c-IAP2 expression **was consistently more intense in malignant NPC cells than in the lymphoid** stroma or in adjacent residual non malignant mucosa.

### The pharmacological agent RMT 5265 induces proteasome-degradation of c-IAP2 and selectively blocks clonogenic growth of NPC cells

The intense expression of c-IAP2 in NPC cells even in a context of hemizygous deletion of *BIRC3* suggested that it was essential for NPC cell growth. To address this hypothesis, it has been used a pharmacological inhibitor of IAP proteins called RMT5265. This compound is a molecular mimic of the N-terminal AVPI sequence of the mature Smac-diablo protein. It is known to bind c-IAP1, c-IAP2 and XIAP [9, 29]. To get formal evidence of its inhibitory effect on c-IAP2, it has been monitored c-IAP2, XIAP and survivin cell-content under treatment by RMT5265. A marked dose-dependent decrease of c-IAP2 - but not XIAP and survivin - was observed in all tested cell types including C666-1 1 NPC cells, Hela cells and NP69 non-malignant nasopharyngeal cells (Figure 2A). The decrease in c-IAP2 content was extremely rapid, maximal in less than one hour (Figure 2B). It was prevented by prior treatment with 2 proteasome inhibitors MG132 and epoxomicin (Eigure 2C). A ladder highly suggestive of ubiquitinylated forms of c-IAP2 was visualized by immunoprecipitation and western blotting in extracts from C666-1 cells. The amount of proteins contained in this ladder was substantially increased when C666-1 cells were treated with RMT5265 for 15 min suggesting that proteasome degradation induced by RMT5265 resulted from enhanced polyubiquitinylation of c-IAP2 (Figure 2D). On the basis of these biochemical data, it has been used RMT5265 to treat NPC and various control cells and assess their clonogenic growth. Although c-IAP2 was degraded by RMT5265 in all cell types, inhibition of clonogenic growth was observed only in NPC cells. A concentration of 50 nM RMT5265 was sufficient to suppress growth of C666-1 cells without influence or very mild effects on non-malignant nasopharyngeal cells (NP69 and NP460).

Clonogenic growth of NPC cells (C666-1) and immortalized nasopharyngeal epithelial cells (NP69 and NP460) treated by RMT5265 shows that cells were seeded at low density in 6-well plates, on a feeder of irradiated Swiss 3T3 fibroblasts as explained in the materials and methods section. Initial cell density: C666-1 cells: 5 000 cells/well; NP69: 500 cells/well; NP460: 2500 cells/well. Colony staining was performed with crystal violet following 2 to 4 weeks of growth. Data are provided in table 4.

**Table 4**

| | untreated | Control agent 100nm | RMT5265 50nm | RMT5265 100nm |
|---|---|---|---|---|
| C666-1 | **∼50 colonies** | **∼50 colonies** | **2 colonies** | **0** |
| NP69 | **∼70 colonies** | **∼70 colonies** | **∼70 colonies** | **∼70 colonies** |
| NP460 | **∼40 colonies** | **∼40 colonies** | **∼30 colonies** | **∼30colonies** |

In a short term colony assay, viability and proliferation of C17 NPC cells was partially decreased by RMT5265 (Figure 3A and 3B).

### Pharmacological or si-RNA inhibition of c-IAP2 induces apoptosis in a fraction of NPC cells.

Next, it has been investigated whether the toxicity of RMT5265 on NPC cells was mediated by apoptosis. When C666-1 cells treated by RMT5265 were investigated by electron microscopy, morphological changes suggestive of caspase-dependent apoptosis, especially strong chromatin compaction, were found in many cells (Figure 4A-Right) [33]. Activation of executionary caspases was confirmed by an *in vitro* chemiluminescent assay on C666-1 cell extracts. In contrast, under the same treatment, caspase activation was almost negligible in non-malignant NP69 cells (Figure 4A-left). Accordingly, the fraction of sub-G1 cells was substantially increased when C666-1 cells - but not NP69 and HeLa - were treated for 48h with concentrations as low as 50 nM of RMT5265. The fraction of C666-1 apoptotic cells was not significantly enhanced by higher concentrations of RMT5265 (200 nM) (Figure 4 B). Although RMT5265 induces selective degradation of c-IAP2, it is also suspected to interfere with XIAP functions [9, 29]. To confirm the anti-apoptotic function of c-IAP2 in the context of NPC cells, it has been used a series of siRNAs specific of c-IAP2, XIAP and Survivin. These siRNAs were selected in order to reduce cross-inhibitory effects to a minimum level (Figure 4C). Extra-cellular LDH release (reflecting global cell death) and activation of caspases-3/7 were assessed in parallel. Inhibition of each IAP induced a significant increase in cell death, supporting the idea that each of them has a distinct, specific and permanent role in NPC cell survival (Figure 4C). Biochemical and morphological characteristics of cell death events triggered by siRNA inhibition were not identical for each IAP. As shown in Figure 4C, for an equivalent level of LDH release, c-IAP2 inhibition produced a lower level of caspase activation than XIAP inhibition. Accordingly, c-IAP2 knock-down resulted in a lower level of DNA fragmentation than XIAP knock-down as shown by a smaller fraction of subG1 cells (Figure 4D).

### Inhibition of c-IAP2 unmasks sensitivity of NPC cells to TLR3-mediated apoptosis.

According to previous studies, several TLRs are frequently expressed by carcinoma cells including TLR3, 4 and 9 [35, 36]. TLR3 and TLR9 transcripts were detected by reverse PCR in all three tested NPC tumor lines (C15, C17 and C666-1) whereas TLR4 was only detected in C15 (Figure 5A). In short term growth assays, C666-1 viability and proliferation was only marginally decreased by the TLR9 agonists, either alone or in combination with RMT5265 (Figure 5B). In contrast, the TLR3 agonist (poly (I:C)) combined to RMT5265 resulted in a very strong cytotoxic effect for C666-1 and C17 cells, even in the range of concentrations of 100 to 500 ng/ml which is relatively low for experimental TLR3 stimulation (Figure 5B an C)[37]. The cytotoxic effect of combined treatment was underlaid by apoptosis and synergistic caspase activation as shown by caspase chemiluminescent assays and PARP cleavage (Figures 5D and 6).

### Apoptosis induced by TLR3 stimulation combined to c-IAP2 inhibition is highly dependent on caspase-8 activation and partially mediated by engagement of TNF-R1.

Previous works performed on other tumor models has shown that apoptosis induction by RMT5265 results from increased TNE-ct production, autocrine stimulation of TNF-R1 and caspase-8-activation dependent on RIPK1 [7-9]. On the other hand TLR3 is known to activate caspase-8 in 293 cells through a TRIF-RIPK1-FADD pathway [38]. Therefore the contribution of caspase-8 and TNF-α to the apoptotic process induced by c-IAP2 inhibition and TLR3 stimulation has been investigated. As shown in Figure 6A, separate treatment by RMT5265 or poly(I:C) induced moderate caspase-8 activation whereas the combination of both agents had a more than additive effect. Using siRNA specific of c-IAP2, XIAP and survivin, only c-IAP2 inhibition induced a significant increase in caspase-8 activation which was additive with the effect of poly(I:C) (Figure 6B). Regarding TNF-α transcription (Figure 6C), it was increased by poly(I:C) but not c-IAP2 inhibition in C666-1 cells in contrast with the ovarian carcinoma Skov 3 used as a positive control. The relative contribution of caspase-8 activation and TNF-R1 stimulation was investigated in the next experiment (Figure 6D). In the absence of any treatment involving c-IAP2 or TLR3, a small level of constitutive caspase-8 activity was detected in C666-1 cells. This basal activity was entirely inhibited either by a caspase-8 blocking peptide or a TNF-R1 blocking peptide. When cells were treated with RMT5265, caspase-8 was significantly activated and a PARP cleavage became apparent. Both processes were entirely inhibited by the caspase-8 inhibitor and only partially by blocking of the TNF-R1. Caspase-8 activation was moderately increased by the TLR3 agonist and this effect was entirely reversed by blocking TNF-R1, a fact which is consistent with the increase in TNF-α transcription by TLR3 stimulation. In the presence of both agents, caspase-8 activity was dramatically increased in association with a strong PARP cleavage and again these effects were only partially antagonized by blocking TNF-R1.

### Discussion

Data of results reveal a specific pattern of IAP expression in NPC cells. c-IAP2 is consistently expressed at a very high level whereas c-IAP1 is barely detectable. This pattern is opposite to that reported in some other tumors, for example oesophageal carcinomas where c-IAPI is overexpressed in a context of low c-IAP2 expression [5]. Moreover, a consistent overexpression of survivin in NPC tumor lines as reported so far for all malignant cells has been found.

The *BIRC3* gene encoding c-IAP2 maps to chromosome 11q22, which is often affected by hemizygous deletion or loss of heterozygoty (LOH) in NPCs, especially at late stages of progression [15, 19]. It has been noticed that even in a context of 11q22 LOH, c-IAP2 remained at a very high level, suggesting that its expression was critical for NPC cell survival or proliferation through all stages of tumor progression. A series of experiments based on c-IAP2 inhibition, using either siRNA interference or the pharmacological agent, RMT5265 has been performed. By itself, RMT5265 inhibited clonogenic growth of NPC cells (Figure 3). Although it cannot exclude a contribution of XIAP inhibition in the growth inhibitory effect of RMT5265, there are two evidences that c-IAP2 is a major target for this drug in NPC cells: 1) regardless of the cellular background, RMT5265 induces a dramatic and very rapid degradation of c-IAP2 (Figure 2); 2) RMT5265 has no significant effects against the non-malignant nasopharyngeal epithelial cells NP69 which have the same amount of XIAP protein than NPC cells and a very low level of c-IAP2 (Figures 1 and 3). In addition, specific inhibition of c-IAP2 by siRNA also has pro-apoptotic effects (Figure 4).

Rapid massive apoptosis of NPC cells was obtained when c-IAP2 inhibition was combined to TLR3 stimulation. A remarkable synergistic effect on caspase-8 activation was achieved by this combined treatment. The resulting activation of caspase-8 was in part mediated by autocrine engagement of TNF-R1 receptors on NPC cells (Figure 6).

Date of present example show that TLR3 stimulation in NPC cells also have apoptosis-inducing effects which are antagonized by c-IAP2. This might explain why on the long term NPC cells are vulnerable to RMT5265 by itself.

Finally, it is noteworthy that various types of pharmacological c-IAP2 inhibitors and TLR3 agonists are currently under therapeutic evaluation [2, 43]. Combination of both types of agents might be beneficial for NPC patients. The results obtained on the SKOV 3 ovarian carcinoma cell line suggest that benefits of this combination may extend to patients suffering from other malignancies (Figure 6 and data not shown).

### REFERENCES

1. Hunter AM, LaCasse EC, Korneluk RG: The inhihitors of apoptosis (IAPs) as cancer targets. Apoptosis 2007, 12:1543-1568.
2. Vucic D, Fairbrother WJ: The inhihitor of apoptosis proteins as therapeutic targets in cancer. Clin Cancer Res 2007, 13:5995-6000.
3. Altieri DC: Survivin, cancer networks and pathway-directed drug discovery. Nat Rev Cancer 2008, 8:61-70.
4. Zender L, Spector MS, Xue W, Flemming P, Cordon-Cardo C, Silke J, Fan ST, Luk JM, Wigler M, Hannon GJ, et al: Identification and validation of oncogenes in liver cancer using an integrative oncogenomic approach. Cell 2006, 125:1253-1267.
5. Imoto I, Yang ZQ, Pimkhaokham A, Tsuda H, Shimada Y, Imamura M, Ohki M, Inazawa J: Identification of cIAPi as a candidate target gene within an amplicon at 11q22 in esophageal squamous cell carcinomas. Cancer Res 2001, 61:6629-6634.
6. Newton K, Vucic D: Uhiquitin ligases in cancer: ushers for degradation. Cancer investigation 2007, 25:502-513.
7. Varfolomeev E, Blankenship JW, Wayson 5M, Fedorova AV, Kayagaki N, Garg P, Zobel K, Dynek JN, Elliott LO, Wallweber RI, et al: IAP Antagonists Induce Autoubiquitination of c-IAPs, NF-kappaB Activation, and TNFalpha-Dependent Apoptosis. Cell 2007, 131:669-681.
8. Vince JE, Wong WW, Khan N, Feltham R, Chau D, Ahmed AU, Benetatos CA, Chunduru 5K, Condon SM, McKinlay M, et al: IAP Antagonists Target cIAP1 to Induce TNFalpha-Dependent Apoptosis. Cell 2007, 131:682-693.
9. Petersen SL, Wang L, Yalcin-Chin A, Li L, Peyton M, Minna J, Harran P, Wang X: Autocrine TNFalpha signaling renders human cancer cells susceptible to Smac-mimetic-induced apoptosis. Cancer Cell 2007, 12:445-456.
10. Busson P, Keryer C, Ooka T, Corbex M: EBV-associated nasopharyngeal carcinomas: from epidemiology to virus-targeting strategies. Trends Microbiol 2004, 12:356-360.
11. Chang ET, Adami HO: The enigmatic epidemiology of nasopharyngeal carcinoma. Cancer Epidemiol BiomarkersPrev2006, 15:1765-1777.
12. Feng BJ, Jalbout M, Ayoub WB, Khyatti M, Dahmoul S, Ayad M, Maachi F, Bedadra W, Abdoun M, Mesli S, et al: Dietary risk factors for nasopharyngeal carcinoma in Maghrebian countries. Int J Cancer 2007, 121:1550-1555.
13. Lu SJ, Day NE, Degos L, Lepage V, Wang PC, Chan SH, Simons M, McKnight B, Easton D, Zeng Y, et al.: Linkage of a nasopharyngeal carcinoma susceptibility locus to the HLA region. Nature 1990, 346:470-471.
14. Samanta M, Iwakiri D, Kanda T, Imaizumi T, Takada K: EB virus-encoded RNAs are recognized by RIG-1 and activate signaling to induce type I IFN. Embo J2006, 25:4207-4214.
15. Lo KW, Huang DP: Genetic and epigenetic changes in nasopharyngeal carcinoma. Semih. Cancer Biol 2002, 12:451-462.
16. Hui AB, Or YY, Takano H, Tsang RK, To KF, Guan XY, Sham JS, Hung KW, Lam CN, van Hasselt CA, et al: Array-based comparative genomic hybridization analysis identified cyclin Dl as a target oncogene at 11q13.3 in nasopharyngeal carcinoma. Cancer Res 2005, 65:8125-8133.
17. Ma BB, Chan AT: Systemic treatment strategies and therapeutic monitoring for advanced nasopharyngeal carcinoma. Expert review of anticancer therapy 2006,6: 383-394.
18. Vicat JM, Ardila-Osorio H, Khabir A, Brezak MC, Viossat I, Kasprzyk P, Jlidi R, Opolon P, Ooka T, Prevost G, et al: Apoptosis and TRAF-1 cleavage in Epstein-Barr virus-positive nasopharyngeal carcinoma cells treated with doxorubicin combined with a farnesyl-transferase inhibitor. Biochem Pharmacol 2003, 65:423-433.
19. Rodriguez SK, A. Keryer, C. Drira, M. Ghorbel, A. Jlidi, R. Bernehiem, A. Valent, A. Busson, P.: Conventional and array-based CGH analysis of Nasopharyngeal Carcinomas from the Mediterranean area: high frequency of gains at 1q and 12p and losses at 11q and 13q. Cancer Genetics and Cytogenetics 2005, 157:343-347.
20. Khabir A, Karray H, Rodriguez S, Rose M, Daoud J, Frikha M, Boudarawa T, Middeldorp J, Jlidi R, Busson P: EBV latent membrane protein 1 abundance correlates with patient age but not with metastatic behavior in north African nasopharyngeal carcinomas. Virol J 2005, 2:39.
21. Hui AB, Cheung ST, Fong Y, Lo KW, Huang DP: Characterization of a new EBV-associated nasopharyngeal carcinoma cell line. Cancer Genet Cytogenet 1998, 101:83-88.
22. Tsao SW, Wang X, Liu Y, Cheung YC, Feng H, Zheng Z, Wong N, Yuen PW, Lo AK, Wong YC, Huang DP: Establishment of two immortalized nasopharyngeal epithelial cell lines using SV4O large T and HPV16E6/E7 viral oncogenes. Biochim Biophys Acta 2002, 1590:150-158.
23. Li HM, Man C, Jin Y, Deng W, Yip YL, Feng HC, Cheung YC, Lo KW, Meltzer P5, Wu ZG, et al: Molecular and cytogenetic changes involved in the immortalization of nasopharyngeal epithelial cells by telomerase. Int J Cancer 2006, 119:1567-1576.
24. Busson P, Zhang Q, Guillon JM, Gregory CD, Young LS, Clausse B, Lipinski M, Rickinson AB, Tursz T: Elevated expression ofICAM1 (CD54) and minimal expression of LFA3 (CD58) in Epstein-Barr-virus-positive nasopharyngeal carcinoma cells. Int J Cancer 1992, 50:863-867.
25. Jacobs JP, Jones CM, Baille JP: Characteristics of a human diploid cell designated MRC-5. Nature 1970, 227:168-170.
26. Sbih-Lammali F, Clausse B, Ardila-Osorio H, Guerry R, Talbot M, Havouis S, Ferradini L, Bosq J, Tursz T, Busson P: Control of apoptosis in Epstein Barr virus-positive nasopharyngeal carcinoma cells: opposite effects of CD95 and CD4O stimulation. Cancer Res 1999, 59:924-930.
27. Cappellen D, Schlange T, Bauer M, Maurer F, Hynes NE: Novel c-MYC target genes mediate differential effects on cell proliferation and migration. EMBO Rep 2007, 8:70-76.
28. Valent A, Benard J, Clausse B, Barrois M, Valteau-Couanet D, Terrier-Lacombe MJ, Spengler B, Bernheim A: In vivo elimination of acentric double minutes containing amplified MYCN from neuroblastoma tumor cells through the formation of micronuclei. Am JPathol 2001, 158:1579-1584.
29. Li L, Thomas RM, Suzuki H, De Brabander JK, Wang X, Harran PG: A small molecule Smac mimic potentiates TRAIL- and TNFalpha-mediated cell death. Science 2004, 305:1471-1474.
30. Rheinwald JG, Green H: Serial cultivation of strains of human epidermal keratinocytes: the formation of keratinizing colonies from single cells. Cell 1975, 6:331-343.
31. Pioche-Durieu C, Keryer C, Souquere S, Bosq J, Faigle W, Loew D, Hirashima M, Nishi N, Middeldorp J, Busson P: In nasopharyngeal carcinoma cells, Epstein-Barr virus LMP interacts with galectin 9 in membrane raft elements resistant to simvastatin. J Viroi200S, 79:13326-13337.
32. Hui AB, Lo KW, Leung 5F, Choi PH, Fong Y, Lee JC, Huang DP: Loss of heterozygosity on the long arm of chromosome ii in nasopharyngeal carcinoma. Cancer Res 1996, 56:3225-3229.
33. Leist M, Jaattela M: Four deaths and a funeral: from caspases to alternative mechanisms. Nat Rev Mol Ceil Bio] 2001, 2:589-598.
34. Conte D, Holcik M, Lefebvre CA, Lacasse E, Picketts DJ, Wright KE, Korneluk RG: Inhibitor of apoptosis protein cIAP2 is essential for lipopolysaccharide-induced macrophage survival. Mol CellBioi2006, 26:699-708.
35. Huang B, Zhao J, Unkeless JC, Feng ZH, Xiong H: TLR signaling by tumor and immune cells: a double-edged sword. Oncogene 2008, 27:218-224.
36. Salaun B, Coste I, Rissoan MC, Lebecque SJ, Renno T: TLR3 can directly trigger apoptosis in human cancer cells. JImmunoI2006, 176:4894-4901.
37. Salaun B, Lebecque S, Matikainen S, Rimoldi D, Romero P: Toll-like receptor 3 expressed by melanoma cells as a target for therapy? Clin Cancer Res 2007, 13:4565-4574.
38. Han KJ, Su X, Xu LG, Bin LH, Zhang J, Shu HB: Mechanisms of the TRIF-induced interferon-stimulated response element and NF-kappaB activation and apoptosis pathways. J Biol Chem 2004, 279:15652-15661.
39. Letai AG: Diagnosing and exploiting cancer's addiction to blocks in apoptosis. Nat Rev Cancer2008, 8:121-132.
40. Weinstein IB, Joe AK: Mechanisms of disease: Oncogene addiction--a rationale for molecular targeting in cancer therapy. Nature clinical practice 2006, 3:448-457.
41. Yip KW, Shi W, Pintilie M, Martin JD, Mocanu JD, Wong D, MacMilian C, Gullane P, O'Sullivan B, Bastianutto C, Liu FF: Prognostic significance of the Epstein-Barr virus, p53, Bci-2, and survivin in nasopharyngeal cancer. Clin. Cancer Res 2006, 12:5726-5732.
42. Iwakiri D, Sheen TS, Chen JY, Huang DP, Takada K: Epstein-Barr virus-encoded small RNA induces insulin-like growth factor 1 and supports growth of nasopharyngeal carcinoma-derived cell lines. Oncogene 2005, 24:1767-1773.
43. Salaun B, Romero P, Lebecque S: Toll-like receptors' two-edged sword: when immunity meets apoptosis. Eur J Immunol 2007, 37:3311-3318.
44. Lu YJ, Williamson D, Clark J, Wang R, Tiffin N, Skelton L, Gordon T, Williams R, Allan B, Jackman A, et al: Comparative expressed sequence hybridization to chromosomes for tumor ciassification and identification of genomic regions of differential gene expression. Proc Natl Acad Sci USA 2001, 98:9197-9202.
45. Belyaev IY and Harms-Ringdahl M., (2002) Radiats Biol Radioecol. 42: 279-83.
46. Edston E. et al., (2002) Int J Legal Med 116:22-6.
47. Wang X et al. (2002) Biochim Biophys Acta 1564: 412-20.
48. Du et al., Cell 102:33-42 (2000).
49. Verhagen et al., Cell 102:43-53 (2000).
50. Bass, Nature 411: 428-29 (2001).
51. Elbahir et al., Nature 411: 494-98 (2001).
52. Fire et al., Nature 391: 806-11 (1998).
53. Guo et al., Blood 99(9):3419-3426 (2002).
54. Srinivasula et al., J. Biol. Chem. 275:36152-36157 (2000).
55. Alexopoulou et al., 2001.
56. Kandimalla et al. ((2003) Nucl. Acid. Res. 31(9): 2393-2400).
57. Gil, J., Alcami, J., and Esteban, M. 1999. Induction of apoptosis by double-stranded-RNA-dependent protein kinase (PKR) involves the alpha subunit of eukaryotic translation initiation factor 2 and NF-kappaB. Mol Cell Biol 19:4653-4663.
58. Alexopoulou, L., Holt, A. C., Medzhitov, R., and Flavell, R. A. 2001. Recognition of double-stranded RNA and activation of NF-kappaB by Toll-like receptor 3. Nature 413:732-738.
59. Field et al.: Proc. Nat. Acad. Sci. U.S. 58, 1004, (1967).
60. Field et al.: Proc. Nat. Acad. Sci. U.S. 58, 2102, (1967).
61. Field et al.: Proc. Nat. Acad. Sci. U.S. 61, 340, (1968).
62. Tytell et al.: Proc. Nat. Acad. Sci. U.S. 58, 1719, (1967).
63. Field et al.: J. Gen. Physiol. 56, 905 (1970).
64. De Clercq et al.: Methods in Enzymology, 78, 291 (1981).

## Claims

1. Products containing:
(i) at least one antagonist of c-IAP2 (cellular inhibitor of apoptosis protein 2), and
(ii) at least one agonist of TLR3 (Toll Like Receptor 3),
as a combined preparation for simultaneous, separate, or sequential use for treating a cancer expressing c-IAP2, in a subject.

2. Products according to claim 1, **characterized in that** cancer expressing c-IAP2 is nasopharyngeal carcinoma (NPC).

3. Products according to claim 1 or 2, **characterized in that** the antagonist of c-IAP2 is selected in the group comprising siRNA directed against the c-IAP2 transcripts, chemical compounds, and peptidic inhibitors of c-IAP2 and combinations thereof.

4. Products according to claim 3, **characterized in that** the peptidic inhibitors of c-IAP2 is as SMAC/diablo protein or mimetic of SMAC/diablo protein.

5. Products according to claim 4, **characterized in that** the mimetic of SMAC/diablo protein is RMT5265.

6. Products according to any of claims 1 to 5, **characterized in that** the agonist of TLR3 is selected in the group comprising single-stranded deoxyribonucleotides, double-stranded deoxyribonucleotides, single-stranded ribonucleotides, double-stranded ribonucleotides and combinations thereof.

7. Products according to claim 6, **characterized in that** the agonist of TLR3 is poly(I:C).

8. Products according to any of claims 1 to 7, **characterized in that** the subject is a mammal, preferably a human.

9. Pharmaceutical composition comprising:
(iv) at least one antagonist of c-IAP2 (cellular inhibitor of apoptosis protein 2), and
(v) at least one agonist of TLR3 (Toll Like Receptor 3), and
(vi) optionally, a pharmaceutically acceptable carrier.

10. Pharmaceutical composition according to claim 9, **characterized in that** the antagonist of c-IAP2 is selected in the group comprising siRNA directed against the c-IAP2 transcripts, chemical compounds, and peptidic inhibitors of c-IAP2 and combinations thereof.

11. Pharmaceutical composition according to claim 9 or 10, **characterized in that** the peptidic inhibitors of c-IAP2 is as SMAC/diablo protein or mimetic of SMAC/diablo protein.

12. Pharmaceutical composition according to claim 11, **characterized in that** the mimetic of SMAC/diablo protein is RMT5265.

13. Pharmaceutical composition according to any of claims 9 to 12, **characterized in that** the agonist of TLR3 is selected in the group comprising single-stranded deoxyribonucleotides, double-stranded deoxyribonucleotides, single-stranded ribonucleotides, double-stranded ribonucleotides and combinations thereof.

14. Pharmaceutical composition according to claim 13, **characterized in that** the agonist of TLR3 is poly(I:C).


SEQUENCE LISTING

<110>  Institut Gustave Roussy (IGR)
       Friboulet, luc
       Busson, Pierre
       Durieu, Catherine

<120>  COMBINATION PRODUCTS FOR TREATING CANCER

<130>  BEP080118

<160>  33

<170>  PatentIn version 3.3

<210>  1
<211>  239
<212>  PRT
<213>  Homo sapiens

<400>  1

<210>  2
<211>  6
<212>  PRT
<213>  Artificial

<220>
<223>  mimetic of SMAC/diablo protein: antagonist of c-IAP2

<400>  2

Ala Val Pro Ile Ala Gln
1               5

<210>  3
<211>  19
<212>  RNA
<213>  Artificial sequence

<220>
<223>  siRNA directed against c-IAP2

<400>  3
aaugcagagu caucaauua                                                    19

<210>  4
<211>  19
<212>  RNA
<213>  Artificial sequence

<220>
<223>  siRNA directed against c-IAP2

<400>  4
ugaucuugug uuagacuua                                                    19

<210>  5
<211>  19
<212>  RNA
<213>  Artificial sequence

<220>
<223>  siRNA directed against c-IAP2

<400>  5
cugccggaau uauuaauga                                                    19

<210>  6
<211>  19
<212>  RNA
<213>  Artificial sequence

<220>

<223>  siRNA directed against c-IAP2

<400>  6
gcaagagaac ugauugaua                                                    19


<210>  7
<211>  19
<212>  RNA
<213>  Artificial sequence

<220>
<223>  siRNA directed against c-IAP2

<400>  7
gaugcaauca ugaugaaua                                                    19


<210>  8
<211>  19
<212>  RNA
<213>  Artificial sequence

<220>
<223>  siRNA directed against c-IAP2

<400>  8
auacgacuug ucaugugaa                                                    19


<210>  9
<211>  19
<212>  RNA
<213>  Artificial sequence

<220>
<223>  siRNA directed against c-IAP2

<400>  9
ggugugcaua uauguugaa                                                    19


<210>  10
<211>  19
<212>  RNA
<213>  Artificial sequence

<220>
<223>  siRNA directed against c-IAP2

<400>  10
ucgaaggugu gcauauaug                                                    19


<210>  11
<211>  19
<212>  RNA
<213>  Artificial sequence

<220>
<223>  siRNA directed against c-IAP2

<400>  11
ggauaaugcu augucagaa                                                    19


<210>  12
<211>  19
<212>  RNA

<213>  Artificial sequence

<220>
<223>  siRNA directed against c-IAP2

<400>  12
ucuaccagug gaagaacaa                                                    19


<210>  13
<211>  19
<212>  RNA
<213>  Artificial sequence

<220>
<223>  siRNA directed against c-IAP2

<400>  13
ucagcuucaa gacacuuca                                                    19


<210>  14
<211>  19
<212>  RNA
<213>  Artificial sequence

<220>
<223>  siRNA directed against c-IAP2

<400>  14
auacuccugu gauuaaugc                                                    19


<210>  15
<211>  19
<212>  RNA
<213>  Artificial sequence

<220>
<223>  siRNA directed against c-IAP2

<400>  15
agaggagaca guccuacug                                                    19


<210>  16
<211>  19
<212>  RNA
<213>  Artificial sequence

<220>
<223>  siRNA directed against c-IAP2

<400>  16
ugcagaguca ucaauuauc                                                    19


<210>  17
<211>  19
<212>  RNA
<213>  Artificial sequence

<220>
<223>  siRNA directed against c-IAP2

<400>  17
gaacaauugc ggagacuac                                                    19

<210>  18
<211>  19
<212>  RNA
<213>  Artificial sequence

<220>
<223>  siRNA directed against c-IAP2

<400>  18
auuccguuaa caacuugga                                                    19


<210>  19
<211>  19
<212>  RNA
<213>  Artificial sequence

<220>
<223>  siRNA directed against c-IAP2

<400>  19
agagaacuga uugauacga                                                    19


<210>  20
<211>  19
<212>  RNA
<213>  Artificial sequence

<220>
<223>  siRNA directed against c-IAP2

<400>  20
ggugugagua cuugauaag                                                    19


<210>  21
<211>  19
<212>  RNA
<213>  Artificial sequence

<220>
<223>  siRNA directed against c-IAP2

<400>  21
uacgacuugu caugugaac                                                    19


<210>  22
<211>  19
<212>  RNA
<213>  Artificial sequence

<220>
<223>  siRNA directed against c-IAP2

<400>  22
gcugaagcug uguuauaug                                                    19


<210>  23
<211>  19
<212>  RNA
<213>  Artificial sequence

<220>
<223>  siRNA directed against c-IAP2

<400>  23

aauuccacac acucauuac                                                    19

<210>  24
<211>  19
<212>  RNA
<213>  Artificial sequence

<220>
<223>  siRNA directed against c-IAP2

<400>  24
uuccguuaac aacuuggaa                                                    19

<210>  25
<211>  19
<212>  RNA
<213>  Artificial sequence

<220>
<223>  siRNA directed against c-IAP2

<400>  25
gaggagacag uccuacuga                                                    19

<210>  26
<211>  19
<212>  RNA
<213>  Artificial sequence

<220>
<223>  siRNA directed against c-IAP2

<400>  26
cagcugcuau ccacaucag                                                    19

<210>  27
<211>  19
<212>  RNA
<213>  Artificial sequence

<220>
<223>  siRNA directed against c-IAP2

<400>  27
caauugcgga gacuacaag                                                    19

<210>  28
<211>  19
<212>  RNA
<213>  Artificial sequence

<220>
<223>  siRNA directed against c-IAP2

<400>  28
uauugcagcc acuguauuc                                                    19

<210>  29
<211>  19
<212>  RNA
<213>  Artificial sequence

<220>

<223>  siRNA directed against c-IAP2

<400>  29
cucucugcaa gaagcugaa                                                    19


<210>  30
<211>  19
<212>  RNA
<213>  Artificial sequence

<220>
<223>  siRNA directed against c-IAP2

<400>  30
ugucagaaca ccugagaca                                                    19


<210>  31
<211>  25
<212>  RNA
<213>  Artificial sequence

<220>
<223>  siRNA HSS100563 directed against c-IAP2

<400>  31
uuuaccaggc uucuacuaaa gccca                                             25


<210>  32
<211>  25
<212>  RNA
<213>  Artificial sequence

<220>
<223>  siRNA HSS100562 directed against c-IAP2

<400>  32
uuaacuagaa cacuagaggg ccagu                                             25


<210>  33
<211>  25
<212>  RNA
<213>  Artificial sequence

<220>
<223>  siRNA HSS100561 directed against c-IAP2

<400>  33
gcugcagauu cguucagagu cuaaa                                             25